# EUROPEAN PATENT SPECIFICATION

(11) **EP 3 119 887 B1**
(45) Date of publication and mention of the grant of the patent: **20.02.2019**
(21) Application number: 15711195.6
(22) Date of filing: 20.03.2015
(51) Int. Cl.: C12N 15/11, C12N 15/113, A61K 31/713

(54) **IMPROVED SMALL INTERFERING RIBONUCLEIC ACID MOLECULES**
VERBESSERTE SMALL-INTERFERING-RIBONUKLEINSÄUREMOLEKÜLE
PETITES MOLÉCULES D'ACIDE RIBONUCLÉIQUE D'INTERFÉRENCE AMÉLIORÉES

(30) Priority: 20.03.2014 SE 1450312
(43) Date of publication of application: 25.01.2017
(73) Proprietor: Varghese, Oommen, 754 46 Uppsala (SE); Oommen, Oommen, 756 46 Uppsala (SE)
(72) Inventor: Varghese, Oommen, 754 46 Uppsala (SE); Oommen, Oommen, 756 46 Uppsala (SE)
(74) Representative: Boult Wade Tennant LLP
(86) International application number: PCT/EP2015/056012
(87) International publication number: WO 2015/140330

(56) References cited:
- EP-A2- 2 631 291
- WO-A1-2006/128739
- WO-A1-2011/075188
- WO-A2-02/44321
- WO-A2-2005/079533
- WO-A2-2010/033247
- ZHONGPING YUAN ET AL: "Asymmetric siRNA: New Strategy to Improve Specificity and Reduce Off-Target Gene Expression", HUMAN GENE THERAPY, vol. 23, no. 5, 9 January 2012 (2012-01-09), pages 521-532, XP055192760, ISSN: 1043-0342, DOI: 10.1089/hum.2011.145
- A.-L. BOLCATO-BELLEMIN ET AL: "Sticky overhangs enhance siRNA-mediated gene silencing", PROCEEDINGS OF THE NATIONAL ACADEMY OF SCIENCES, vol. 104, no. 41, 9 October 2007 (2007-10-09), pages 16050-16055, XP055050168, ISSN: 0027-8424, DOI: 10.1073/pnas.0707831104
- FREDERICO PITTELLA ET AL: "Enhanced endosomal escape of siRNA-incorporating hybrid nanoparticles from calcium phosphate and PEG-block charge-conversional polymer for efficient gene knockdown with negligible cytotoxicity", BIOMATERIALS, ELSEVIER SCIENCE PUBLISHERS BV., BARKING, GB, vol. 32, no. 11, 31 December 2010 (2010-12-31), pages 3106-3114, XP028145194, ISSN: 0142-9612, DOI: 10.1016/J.BIOMATERIALS.2010.12.057 [retrieved on 2011-01-11]
- LEI LV ET AL: "Silencing USP22 by asymmetric structure of interfering RNA inhibits proliferation and induces cell cycle arrest in bladder cancer cells", MOLECULAR AND CELLULAR BIOCHEMISTRY, KLUWER ACADEMIC PUBLISHERS, BO, vol. 346, no. 1 - 2, 9 September 2010 (2010-09-09), pages 11-21, XP019866356, ISSN: 1573-4919, DOI: 10.1007/S11010-010-0585-4

## Description

### TECHNICAL FIELD

The present embodiments generally relate to small interfering RNA (siRNA) molecules, and in particular to siRNA molecules having improved characteristics in terms of cell penetration.

### BACKGROUND

Since the discovery of synthetic small interfering RNA (siRNA), sometimes denoted short interfering RNA or silencing RNA in the art, as a potent gene silencing molecule in mammalian cells, siRNA based drugs have emerged as a promising technology to treat a variety of diseases. RNA interference (RNAi) is an endogeneous pathway for post-transciptional gene silencing that utilizes sequence specific knockdown of target mRNA sequences, making RNAi more specific than traditional drugs. In this RNAi technology, short double-stranded RNA (19-mer to 21-mer) is incorporated into an RNA-induced silencing complex (RISC), which results in degradation of one strand, denoted sense or passenger strand, and incorporation of the other strand, denoted antisense or guide strand, into the RISC complex. This activated RISC complex binds mRNA in a sequence specific manner, resulting in knockdown of gene expression. The potency of this method has been so profound that it outperforms other existing gene silencing technologies.

In order to bring the RNAi technology for clinical translation, several challenges need to be addressed. The key aspects being (1) cellular delivery of such therapeutic siRNA molecules at desired concentrations; (2) selective incorporation of antisense strand in the RISC complex to minimize undesired off-target effects; and (3) to minimize immune activation.

It is well established that siRNA, being a large negatively charged molecule, generally does not pass the plasma membrane of cells, the biological barrier that limits its therapeutic potential. Thus, extensive research is being conducted to identify delivery carriers that overcome this cellular barrier without inducing significant toxicity. Naked siRNA can induce therapeutic effect *in vivo*, albeit, requiring high-pressure (so-called hydrodynamic) intravenous injection to force the siRNA molecules into cells. Localized delivery of naked siRNA is still pursued as an attractive alternative, especially in the eye where large number of clinical trials is being conducted.

The second major challenge remains preferential selection of desired antisense strand over sense strand within the RNAi machinery. Specific proteins in the RISC complex selectively bind thermodynamically more stable 5'-end of siRNA, thereby regulating this selection process. To improve antisense strand selection, several approaches have been pursued, such as development of asymmetric RNA by reducing the length of the sense strand, regulating thermodynamic asymmetry by selective incorporation of destabilizing chemical modification, such as unlocked nucleic acids, internally destabilized duplexes and by blocking phosphorylation of 5'-end of sense strand using 5'-O-methyl modifications.

US 2009/0208564 describes design of asymmetric siRNA molecules where the length of sense or passenger strand is reduced from conventional 21-mer to 12-17-mer. Such asymmetry reduces off-target effect and improves RNAi activity.

EP 1 407 044 (WO0244321) describes isolated dsRNA molecules capable of target-specific RNA interference, in which each RNA strand has a length from 19 to 23 nucleotides and wherein at least one strand has a 3'-overhang from 1 to 3 nucleotides. The dsRNA molecules of EP 1 407 044 require the presence of a carrier for transfection.

EP 1486 564 describes a method for the specific selection of dsRNA molecules capable of RNA interference and having improved efficiency through increased serum stability. The sequences of the single strands of the dsRNA molecule are selected such that at each end of the dsRNA molecule the last complementary nucleotide pair is G-C or at least two of the last four complementary nucleotide pairs are G-C pairs, wherein the dsRNA molecule has an overhang of 1-4 unpaired nucleotides.

US 2012/0041049 describes isolated dsRNA molecules possessing G/C rich sequences at the 5'-end of the passenger strand or at the 3'-end of the guide strand resulting in enhanced serum stability and knockdown efficiency.

The only carrier free delivery of siRNA is described in US2004/0198640, US 2009/0209626 and WO 2010/033247, where nucleotides of the siRNA molecules are extensively modified with hydrophobic groups.

Other siRNA molecules are described in Caplan et al., PNAS, 98, 17, 9742-9747 (2001); Kim et al., Nature Biotechnology, 23, 2, 222-226 (2005); Bolcato-Bellmin et al., PNAS, 104, 41, 16050-16055 (2007); Mok et al., Nature Materials, 9, 272-278 (2010); Schmitz and Chu, Silence, 2, 1, 1-10 (2011); Wu et al., Nucleic Acids Research, 1-10 (2013); Zhongping et al., Human Gene Therapy, 23, 5, 521-532 (2012); WO03/012052; WO2005/014782; WO2005/079533; WO2006/128739; WO2010/080129; and WO 2011/072082.

A major area of research within the field of RNAi technology is to identify the most biocompatible transfection reagent. The technical problem is the inevitable need of carrier systems like cationic polymers, virus particles or hydrophobic modifications of nucleotides for cellular delivery. Till today, there are no reports of cellular delivery of bioactive RNAi molecules that do not require a complexing agent or other external stimuli as transfection agent.

### SUMMARY

It is a general objective to provide an improved double-stranded small interfering ribonucleic acid (siRNA) molecule.

It is a particular objective to provide a double-stranded siRNA molecule having a design that promotes cellular uptake without the need for any transfection agent.

These and other objectives are met by embodiments as defined herein.

The invention provides a double-stranded small interfering ribonucleic acid (siRNA) molecule comprising a sense RNA strand and an antisense RNA strand, wherein said sense strand has a 3'-overhang of from four to eight nucleotides, of which at least one nucleotide is a non-ribonucleotide, wherein said antisense strand has a 3'-overhang that is shorter than said 3'-overhang of said sense strand, and wherein a double-stranded portion of said double-stranded siRNA molecule has a length of at least 19 base pairs.

The disclosure provides a double-stranded small interfering ribonucleic acid (siRNA) molecule. The double-stranded siRNA molecule may comprise a sense RNA strand and an antisense RNA strand, wherein the sense strand has a 3'-overhang of from 4 to 8 nucleotides. The antisense strand may have a 3'-overhang that is shorter than the 3'overhang of the sense strand.

The double-stranded siRNA molecule may be administered and taken up by cells without the use of any other agent or reagent. The double-stranded siRNA molecules may be taken up by cells in the absence of a transfection agent, reagent and/or vector. The double-stranded siRNA molecules may be taken up by cells with or without the use of a carrier. The double-stranded siRNA molecules may be taken up by cells in the absence of any hydrophobic modification of the double-stranded siRNA molecule.

The double-stranded small interfering ribonucleic acid (siRNA) molecule may comprise a sense RNA strand and an antisense RNA strand, wherein the sense strand has a 3'-overhang of from 4 to 8 nucleotides, of which at least one nucleotide is a non-ribonucleotide (e.g. a deoxynucleotide), and wherein the antisense strand has a 3'-overhang that is shorter than said 3'-overhang of the sense strand.

The double-stranded small interfering ribonucleic acid (siRNA) molecule may comprise a sense RNA strand and an antisense RNA strand, wherein said sense strand has a 3'-overhang of from 4 to 8 nucleotides, of which at least one nucleotide is a non-ribonucleotide (e.g. a deoxynucleotide), and wherein said antisense strand has a 3'-overhang of 2 nucleotides.

The sense strand may have a 3'-overhang of 4 to 8 nucleotides, 4 to 7 nucleotides, 4 to 6 nucleotides, 4 to 5 nucleotides, 5 to 8 nucleotides, 5 to 7 nucleotides, 5 to 6 nucleotides, 6 to 8 nucleotides, 6 to 7 nucleotides, or 7 to 8 nucleotides. Preferably, the sense strand has a 3'-overhang of 4, 5, 6, 7 or 8 nucleotides. Most preferably, the sense strand has a 3'-overhang of 5 nucleotides.

The antisense strand may have a 3'-overhang of at least 2 nucleotides, 2 to 8 nucleotides, 2 to 7 nucleotides, 2 to 6 nucleotides, 2 to 5 nucleotides, 2 to 4 nucleotides, 2 to 3 nucleotides, 3 to 8 nucleotides, 3 to 7 nucleotides, 3 to 6 nucleotides, 3 to 5 nucleotides, 3 to 4 nucleotides, 4 to 8 nucleotides, 4 to 7 nucleotides, 4 to 6 nucleotides, 4 to 5 nucleotides, 5 to 8 nucleotides, 6 to 8 nucleotides, 6 to 7 nucleotides, or 7 to 8 nucleotides. Preferably, the antisense strand has a 3'- overhang of 2, 3, 4, 5, 6, 7 or 8 nucleotides. Most preferably, the antisense strand has a 3'-overhang of 2 nucleotides.

The antisense strand of the double-stranded siRNA molecule may not have a 3'-overhang but rather has a blunt end.

The skilled person will appreciate that the 3'-overhangs of the sense and antisense strands may be any combination of the lengths of overhang described above. Examples of preferred combinations are provided in Table 1 below:

**Table 1**

| Length of sense strand 3' overhang (nucleotides) | Length of antisense strand 3' overhang (nucleotides) |
|---|---|
| 4-8 | 2-5 |
| 4-8 | 2-4 |
| 4-8 | 2-3 |
| 4-8 | 2 |
| 5-8 | 2-5 |
| 5-8 | 2-4 |
| 5-8 | 2-3 |
| 5-8 | 2 |
| 4-7 | 2-5 |
| 4-7 | 2-4 |
| 4-7 | 2-3 |
| 4-7 | 2 |
| 5-7 | 2-5 |
| 5-7 | 2-4 |
| 5-7 | 2-3 |
| 5-7 | 2 |
| 4-6 | 2-5 |
| 4-6 | 2-4 |
| 4-6 | 2-3 |
| 4-6 | 2 |
| 5-6 | 2-5 |
| 5-6 | 2-4 |
| 5-6 | 2-3 |
| 5-6 | 2 |
| 4-5 | 2-4 |
| 4-5 | 2-3 |
| 4-5 | 2 |
| 5 | 2-5 |
| 5 | 2-4 |
| 5 | 2-3 |
| 5 | 2 |
| 6 | 2-5 |
| 6 | 2-4 |
| 6 | 2-3 |
| 6 | 2 |

The sense strand 3'-overhang and/or the antisense strand 3'-overhang may comprise one or more nucleotides that are not ribonucleotides (i.e. non-ribonucleotides). The sense strand 3'-overhang may comprise at least 1, 2, 3, 4, 5, 6, 7 or 8 non-ribonucleotides. Preferably, all of the nucleotides in the sense strand 3'-overhang are non-ribonucleotides. The antisense strand 3'-overhang may comprise at least 1, 2, 3, 4, 5, 6, 7 or 8 non-ribonucleotides. Preferably, all of the nucleotides in the antisense strand 3'-overhang are non-ribonucleotides.

The one or more non-ribonucleotides in the sense strand 3'-overhang and/or the antisense strand 3' overhang may include one or more deoxynucleotides. The one or more deoxynucleotides may be any deoxynucleotide or combination of at least two deoxynucleotides, such as deoxythymidine (dT), deoxyadenosine (dA), deoxyguanosine (dG) or deoxycytosine (dC).

All of the nucleotides of the 3'-overhang of the sense and/or antisense strand may be deoxynucleotides. For example, all deoxynucleotides could be dT, all could be dA, all could be dG or all could be dC. Alternatively, all deoxynucleotides could be purine deoxynucleotides, i.e. dG and/or dA, or all could be pyrimidine deoxynucleotides, i.e. dT and/or dC. Alternatively, a mixture of purine and pyrimidine deoxynucleotides could be used, such as a mixture of dA, dG, dT and/or dC.

Examples of 3'-overhangs for the sense strand include: dT₄, dA₄, dG₄, dC₄, dT₅, dA₅, dG₅, dC₅, dT₆, dA₆, dG₆, dC₆, dT₇, dA₇, dG₇, dC₇, dT₈, dA₈, dG₈ and dC₈; preferably dT₅, dA₅, dG₅, dC₅, dT₆, dA₆, dG₆, dC₆, dT₇, dA₇, dG₇, dC₇, dT₈, dA₈, dG₈ and dC₈; preferably dT₅, dA₅, dG₅, dC₅, dT₆, dA₆, dG₆, dC₆, dT₇, dA₇, dG₇ and dC₇; preferably dT₅, dA₅, dG₅, dC₅, dT₆, dA₆, dG₆ and dC₆; or preferably dT₅, dA₅, dG₅ and dC₅. Alternatively, a combination of different deoxynucleotides may be included in the 3'-overhang of the sense strand.

Examples of 3'-overhangs for the antisense strand include: dT₂, dA₂, dG₂, dC₂, dT₃, dA₃, dG₃, dC₃, dT₄, dA₄, dG₄, dC₄, dT₅, dA₅, dG₅ and dC₅; preferably dT₂, dA₂, dG₂, dC₂, dT₅, dA₅, dG₅ and dC₅; or preferably dT₂, dA₂, dG₂, dC₂. Alternatively, a combination of different deoxynucleotides may be included in the 3'-overhang of the antisense strand.

The skilled person will appreciate that the 3'-overhangs of the sense and antisense strands may be any combination of the overhangs described above. Examples of preferred combinations are provided in Table 2 below:

**Table 2**

| Sense strand 3' overhang | Antisense strand 3' overhang |
|---|---|
| dT₅, dA₅, dG₅ or dC₅ | dT₅, U₅, dT₂ or U₂ |
| dT₅, dA₅, dG₅ or dC₅ | dT₅ or U₅ |
| dT₅, dA₅, dG₅ or dC₅ | dT₂ or U₂ |
| dT₅, dA₅, dG₅ or dC₅ | dT₂ |
| dT₅, dA₅, dG₅ or dC₅ | No overhang |
| dT₅ or dA₅ | dT₅, U₅, dT₂ or U₂ |
| dT₅ or d A₅ | dT₅ or U₅ |
| dT₅ or dA₅ | dT₂ or U₂ |
| dT₅ or dA₅ | dT₂ |
| dT₅ or dA₅ | No overhang |
| dT₅ | dT₅, U₅, dT₂ or U₂ |
| dT₅ | dT₅ or U₅ |
| dT₅ | dT₂ or U₂ |
| dT₅ | dT₂ |
| dT₅ | No overhang |
| dT₅ or dA₅ | dT₄, dT₃, dT₂ or dT |
| dT₅ or dA₅ | dA₄, dA₃, dA₂ or dA |
| dT₅ or dA₅ | U₅, U₄, U₃, U₂ or U |
| dT₅ or dA₅ | dC₄, dC₃, dC₂ or dC |
| dT₅ or dA₅ | dG₄, dG₃, dG₂ or dG |

Alternatively, the one or more non-ribonucleotides in the 3'-overhang of the sense and/or antisense strand 3' overhang may include one or more modified nucleotides, for example one or more dideoxynucleotides, or one or more other modified nucleotides. Modified nucleotides may have modifications on the ribose sugar, the phosphate backbone and/or nucleobase. Non-limiting examples of ribose modifications are analogues to modifications where 2'-OH is replaced by H, SH, SR, R, OR, CI, Br, I, F, CN, NH₂, NHR, NR₂, guanidine, wherein R is an optionally substituted aryl group, C1-C6 alkyl, C2-C6-alkenyl or C2-C6 alkynyl group. Further nucleotide analogues and nucleotide modifications that can be used according to the embodiments are disclosed in paragraph [0017] of EP 1 407 044. The double-stranded siRNA molecule may or may not comprise an orthoester-modified nucleotide.

One or more of the nucleotides of the 3'-overhang of the sense strand and/or antisense may be ribonucleotides. The ribonucleotide(s) may be riboadenosine (rA), riboguanosine (rG), ribouracil (rU) and/or ribocytosine (rC).

The nucleotides of the double-stranded siRNA molecule may or may not be modified with hydrophobic groups (e.g. cholesterol). The double-stranded siRNA molecule may or may not be conjugated to one or more other molecules.

The double-stranded siRNA molecule may have a double-stranded portion with a length of at least 19 base pairs. The double-stranded region may be 19 to 30 base pairs, 19 to 27 base pairs, 19 to 24 base pairs, or 19 to 21 base pairs. For example, the double-stranded region may be 19, 20, 21, 22, 23, 24, 25, 26, 27, 28 or 29 base pairs.

If the double-stranded siRNA molecule has a double-stranded portion of 19 base pairs, the sense strand preferably has a total length of 23 to 27 nucleotides. The antisense strand may then have a total length of 19 nucleotides if having a blunt end or preferably from 21 to 24 nucleotides, such as 21 nucleotides, with a 3'-overhang of two to five, such as two, nucleotides. Preferably, the double-stranded portion is 19 base pairs, the sense strand is 24 base pairs and the antisense strand is 21 base pairs.

The double-stranded portion of the siRNA molecule may or may not comprise one or more mismatches between a nucleotide on the sense strand and the opposite nucleotide on the antisense strand.

The invention further provides a cell transfection method comprising contacting (in vitro) a cell to be transfected with a double-stranded small interfering ribonucleic acid (siRNA) molecule of the invention.

The step of contacting may comprise contacting the cell to be transfected by the double-stranded siRNA molecule in the absence of a transfection agent. The step of contacting may comprise contacting the cell to be transfected by the double-stranded siRNA molecule in the absence of a transfection reagent. The step of contacting may comprise contacting the cell to be transfected by the double-stranded siRNA molecule in the absence of a reagent that facilitates entry of siRNA into a cell and/or release of the siRNA from the endosome into the cytosol. The step of contacting may comprise contacting the cell to be transfected by the double-stranded siRNA molecule in the absence of a cationic agent. The step of contacting may comprise contacting the cell to be transfected by the double-stranded siRNA molecule in the absence of a cationic cell penetrating peptide (CPP); a cationic polymer or dendrimer e.g. polyethylenimine (PEI) or poly-D,L-lactide-co-glycolide (PLGA); and/or a cationic lipid (e.g. lipofectamine).

The invention provides a method of target-specific ribonucleic acid (RNA) interference in a cell comprising contacting in vitro said cell with a double-stranded small interfering RNA (siRNA) molecule in the absence of a cationic agent, wherein said double-stranded siRNA molecule comprises a sense RNA strand and an antisense RNA strand, wherein said sense strand has a 3'-overhang of from four to eight nucleotides, and wherein at least a portion of said antisense strand of said double stranded siRNA molecule has a nucleotide sequence that is complementary to a nucleotide sequence of a target RNA sequence.

The invention further provides a method of target-specific ribonucleic acid (RNA) interference in a cell comprising contacting in vitro the cell with a double-stranded siRNA molecule of the invention, wherein at least a portion of an antisense strand of said double-stranded siRNA molecule has a nucleotide sequence that is complementary to a nucleotide sequence of a target RNA sequence.

The step of contacting may comprise contacting the cell to be transfected by the double-stranded siRNA molecule in the presence or absence of a carrier e.g. an agent or a formulation. Preferably, the carrier promotes accumulation of the double-stranded siRNA molecule at a target site and/or protects the double-stranded siRNA molecule from undesirable interactions with biological milieu components and/or protects the double-stranded siRNA molecule from metabolism and/or degradation.

The carrier may be a viral carrier or a non-viral carrier. Viral carriers include a lentiviral vector or an adenoviral vector for delivery of a DNA-based construct encoding the double-stranded siRNA molecule. Non-viral siRNA carriers (or vectors) include complexing the double-stranded siRNA molecule with a cationic agent such as a cationic cell penetrating peptide (CPP); a cationic polymer or dendrimer e.g. polyethylenimine (PEI) and poly-D,L-lactide-co-glycolide (PLGA); and/or a cationic lipid (e.g. lipofectamine). Preferably, the carrier is not a cationic agent. Preferably the carrier is not a cationic cell penetrating peptide (CPP); a cationic polymer or dendrimer e.g. polyethylenimine (PEI) and poly-D,L-lactide-co-glycolide (PLGA); and/or a cationic lipid (e.g. lipofectamine).

The carrier may be a small molecule (e.g., cholesterol, bile acid, and/or lipid), polymer, protein (e.g. an antibody), and/or aptamer (e.g. RNA) that is conjugated to the double-stranded siRNA molecule. The carrier may be a nanoparticulate formulation used to encapsulate the double-stranded siRNA molecule.

The carrier may be a modification of the double-stranded siRNA molecule with a targeting ligand (e.g. an antibody), a peptide, a small molecule (e.g. folic acid and/or biotin), or a polymer present in extracellular matrix (e.g. hyaluronic acid and/or chondroitin sulphate), or a hydrophobic modification of the double-stranded siRNA molecule (e.g. using cholesterol and/or α-tocopherol). Preferably, the carrier is not a hydrophobic modification of the double-stranded siRNA molecule (e.g. using cholesterol and/or α-tocopherol).

The sequence of the antisense strand of the double-stranded siRNA molecule is selected, in the double-stranded portion or at least a portion thereof, to be complementary to and capable of hybridizing to a target RNA or DNA sequence, preferably a target mRNA sequence. The sequence of the sense strand is selected to be complementary to the antisense strand and form base pairs between the nucleotides in the respective strands in the double-stranded portion of the double-stranded siRNA molecule.

The sequence of the double-stranded siRNA molecule preferably has a sufficient identity to a target nucleotide sequence in order to mediate target-specific RNAi. Preferably, the sequence of the double-stranded portion has an identity to the desired target nucleotide sequence of at least 50%, at least 70%, at least 85 %, at least 90 %, at least 95 %, at least 96%, at least 97%, at least 98%, at least 99%, and most preferably 100%. Preferably, the sequence of the double-stranded portion has identity to the desired target nucleotide sequence over at least 10, 11, 12, 13, 14, 15, 16, 17, 18, 19, 20, 21, 22, 23, 24, 25, 26, 27, 28, 29 or 30 (contiguous) nucleotides.

The invention further provides a double-stranded siRNA molecule of the invention for use as a medicament.

The invention provides a double-stranded small interfering ribonucleic acid (siRNA) molecule for use in the treatment of a disease by sequence-specific knockdown of a target RNA sequence, wherein said double-stranded siRNA molecule comprises a sense RNA strand and an antisense RNA strand, wherein said sense strand has a 3'-overhang of from four to eight nucleotides, wherein at least a portion of an antisense strand of said double-stranded siRNA molecule has a nucleotide sequence that is complementary to a nucleotide sequence of said target RNA sequence, and wherein said double-stranded siRNA molecule is administered to a subject in the absence of a cationic agent.

The invention further provides a double-stranded siRNA molecule of the invention for use in treating a disease by sequence-specific knockdown of a target RNA sequence, wherein at least a portion of an antisense strand of said double-stranded siRNA molecule has a nucleotide sequence that is complementary to a nucleotide sequence of said target RNA sequence.

The sequence of the antisense strand is selected, in the double-stranded portion or at least a portion thereof, to be complementary to and capable of hybridizing to a target RNA or DNA sequence, preferably a target mRNA sequence. The sequence of the sense strand is selected to be complementary to the antisense strand and form base pairs between the nucleotides in the respective strands in the double-stranded portion of the double-stranded siRNA molecule.

The sequence of the double-stranded siRNA molecule preferably has a sufficient identity to a target nucleotide sequence in order to mediate target-specific RNAi. Preferably, the sequence of the double-stranded portion has an identity to the desired target nucleotide sequence of at least 50%, at least 70%, at least 85 %, at least 90 %, at least 95 %, at least 96%, at least 97%, at least 98%, at least 99%, and most preferably 100%. Preferably, the sequence of the double-stranded portion has identity to the desired target nucleotide sequence over at least 10, 11, 12, 13, 14, 15, 16, 17, 18, 19, 20, 21, 22, 23, 24, 25, 26, 27, 28, 29 or 30 (contiguous) nucleotides.

The double-stranded siRNA molecule may be used to treat one or more of the diseases and/or disorders selected from genetic disorders, cancer (e.g. by silencing genes differentially upregulated in tumour cells and/or genes involved in cell division), HIV, other viral infections (e.g. infection caused by hepatitis A, hepatitis B, herpes simplex virus type 2, influenza, measles and/or respiratory syncytial virus), neurodegenerative diseases (e.g. Parkinson's disease and/or polyglutamine diseases such as Huntington's disease), ocular diseases (e.g. macular degeneration) and liver failure.
Potential antiviral therapies using the double-stranded siRNA molecule include one or more of the following: topical microbicide treatment to treat infection by herpes simplex virus type 2, inhibition of viral gene expression in cancerous cells, knockdown of host receptors and/or co-receptors for HIV, silencing of hepatitis A and/or hepatitis B genes, silencing of influenza gene expression, and inhibition of measles viral replication. Potential treatments for neurodegenerative diseases include treatment of polyglutamine diseases such as Huntington's disease.
A subject treated with the double-stranded siRNA molecule may receive the double-stranded siRNA molecule in combination with other forms of treatment for the disorder concerned, including treatment with drugs generally used for the treatment of the disorder. The drugs may be administered in one or several dosage units.
The invention further provides a pharmaceutical composition comprising a double-stranded siRNA molecule of the invention and a pharmaceutically acceptable diluent. The pharmaceutically acceptable diluent may be saline, a buffered solution (e.g. a buffered aqueous solution) or another excipient.

The disclosure further provides a cell transfected with a double-stranded siRNA molecule of the invention or a nucleotide sequence encoding said double-stranded siRNA molecule.
The invention further provides a cell transfection composition comprising a double-stranded siRNA molecule, wherein the double-stranded siRNA molecule comprises a sense RNA strand and an antisense RNA strand, wherein the sense strand has a 3'-overhang of from four to eight nucleotides and wherein the composition does not comprise a cationic agent. The composition may not comprise an agent for facilitating release of said siRNA from the endosome into the cytosol of a cell. The composition may not comprise an agent for facilitating entry of the siRNA into a cell. The composition may not comprise a transfection reagent. The composition may not comprise a cationic cell penetrating peptide (CPP); a cationic polymer or dendrimer e.g. polyethylenimine (PEI) or poly-D,L-lactide-co-glycolide (PLGA); and/or a cationic lipid (e.g. lipofectamine).
The cell transfection composition may or may not comprise a carrier e.g. an agent or a formulation. Preferably, the carrier promotes accumulation of the double-stranded siRNA molecule at a target site and/or protects the double-stranded siRNA molecule from undesirable interactions with biological milieu components and/or protects the double-stranded siRNA molecule from metabolism and/or degradation.

The carrier may be a viral carrier or a non-viral carrier. Viral carriers include a lentiviral vector or an adenoviral vector for delivery of a DNA-based construct encoding the double-stranded siRNA molecule. Non-viral siRNA carriers (or vectors) include complexing the double-stranded siRNA molecule with a cationic agent such as a cationic cell penetrating peptide (CPP); a cationic polymer or dendrimer e.g. polyethylenimine (PEI) and poly-D,L-lactide-co-glycolide (PLGA); and/or a cationic lipid (e.g. lipofectamine). Preferably, the carrier is not a cationic agent. Preferably the carrier is not a cationic cell penetrating peptide (CPP); a cationic polymer or dendrimer e.g. polyethylenimine (PEI) and poly-D,L-lactide-co-glycolide (PLGA); and/or a cationic lipid (e.g. lipofectamine).

The carrier may be a small molecule (e.g., cholesterol, bile acid, and/or lipid), polymer, protein (e.g. an antibody), and/or aptamer (e.g. RNA) that is conjugated to the double-stranded siRNA molecule. The carrier may be a nanoparticulate formulation used to encapsulate the double-stranded siRNA molecule.

The carrier may be a modification of the double-stranded siRNA molecule with a targeting ligand (e.g. an antibody), a peptide, a small molecule (e.g. folic acid and/or biotin), or a polymer present in extracellular matrix (e.g. hyaluronic acid and/or chondroitin sulphate), or a hydrophobic modification of the double-stranded siRNA molecule (e.g. using cholesterol and/or α-tocopherol). Preferably, the carrier is not a hydrophobic modification of the double-stranded siRNA molecule (e.g. using cholesterol and/or α-tocopherol).

The antisense strand of the double-stranded siRNA may have a 3'-overhang that is shorter than said 3'overhang of the sense strand. The double-stranded siRNA molecule may be any double-stranded siRNA molecule of the invention.

The disclosure further provides a pharmaceutical composition comprising a double-stranded siRNA molecule and a pharmaceutically acceptable diluent, wherein the double-stranded siRNA molecule comprises a sense RNA strand and an antisense RNA strand, wherein the sense strand has a 3'-overhang of from four to eight nucleotides. The pharmaceutical composition may not comprise an agent for facilitating release of the siRNA from the endosome into the cytosol of a cell. The pharmaceutical composition may not comprise an agent for facilitating entry of the siRNA into a cell. The pharmaceutical composition may not comprise a cationic agent. The pharmaceutical composition may not comprise a cationic cell penetrating peptide (CPP); a cationic polymer or dendrimer e.g. polyethylenimine (PEI) or poly-D,L-lactide-co-glycolide (PLGA); and/or a cationic lipid (e.g. lipofectamine).

The pharmaceutical composition may or may not comprise a carrier e.g. an agent or a formulation. Preferably, the carrier promotes accumulation of the double-stranded siRNA molecule at a target site and/or protects the double-stranded siRNA molecule from undesirable interactions with biological milieu components and/or protects the double-stranded siRNA molecule from metabolism and/or degradation.

The carrier may be a viral carrier or a non-viral carrier. Viral carriers include a lentiviral vector or an adenoviral vector for delivery of a DNA-based construct encoding the double-stranded siRNA molecule. Non-viral siRNA carriers (or vectors) include complexing the double-stranded siRNA molecule with a cationic agent such as a cationic cell penetrating peptide (CPP); a cationic polymer or dendrimer e.g. polyethylenimine (PEI) and poly-D,L-lactide-co-glycolide (PLGA); and/or a cationic lipid (e.g. lipofectamine). Preferably, the carrier is not a cationic agent. Preferably the carrier is not a cationic cell penetrating peptide (CPP); a cationic polymer or dendrimer e.g. polyethylenimine (PEI) and poly-D,L-lactide-co-glycolide (PLGA); and/or a cationic lipid (e.g. lipofectamine).

The carrier may be a small molecule (e.g., cholesterol, bile acid, and/or lipid), polymer, protein (e.g. an antibody), and/or aptamer (e.g. RNA) that is conjugated to the double-stranded siRNA molecule. The carrier may be a nanoparticulate formulation used to encapsulate the double-stranded siRNA molecule.

The carrier may be a modification of the double-stranded siRNA molecule with a targeting ligand (e.g. an antibody), a peptide, a small molecule (e.g. folic acid and/or biotin), or a polymer present in extracellular matrix (e.g. hyaluronic acid and/or chondroitin sulphate), or a hydrophobic modification of the double-stranded siRNA molecule (e.g. using cholesterol and/or α-tocopherol). Preferably, the carrier is not a hydrophobic modification of the double-stranded siRNA molecule (e.g. using cholesterol and/or α-tocopherol).

The pharmaceutically acceptable diluent may be saline, a buffered solution (e.g. a buffered aqueous solution) or another excipient.

The antisense strand of the double-stranded siRNA may have a 3'-overhang that is shorter than said 3'overhang of said sense strand. The double-stranded siRNA molecule may be any double-stranded siRNA molecule of the invention.

The disclosure further provides a cell transfection method comprising contacting (in vitro) a cell to be transfected with a double-stranded siRNA molecule, wherein the double-stranded siRNA molecule comprises a sense RNA strand and an antisense RNA strand, wherein the sense strand has a 3'-overhang of from four to eight nucleotides, and wherein the double-stranded siRNA molecule is transfected into the cytosol of said cell. The step of contacting (in vitro) the cell to be transfected with a double-stranded siRNA molecule may be performed in the absence of an agent for facilitating release of the siRNA from the endosome into the cytosol of said cell. The step of contacting (in vitro) the cell to be transfected with a double-stranded siRNA molecule may be performed in the absence of an agent for facilitating entry of the siRNA molecule into the cell. The step of contacting (in vitro) the cell to be transfected with a double-stranded siRNA molecule may be performed in the absence of a cationic agent. The step of contacting (in vitro) the cell to be transfected with a double-stranded siRNA molecule may be performed in the absence of a cationic cell penetrating peptide (CPP); a cationic polymer or dendrimer e.g. polyethylenimine (PEI) or poly-D,L-lactide-co-glycolide (PLGA); and/or a cationic lipid (e.g. lipofectamine).

The cell may be contacted with the double-stranded siRNA molecule in the presence or absence of a carrier e.g. an agent or a formulation. Preferably, the carrier promotes accumulation of the double-stranded siRNA molecule at a target site and/or protects the double-stranded siRNA molecule from undesirable interactions with biological milieu components and/or protects the double-stranded siRNA molecule from metabolism and/or degradation.

The carrier may be a viral carrier or a non-viral carrier. Viral carriers include a lentiviral vector or an adenoviral vector for delivery of a DNA-based construct encoding the double-stranded siRNA molecule. Non-viral siRNA carriers (or vectors) include complexing the double-stranded siRNA molecule with a cationic agent such as a cationic cell penetrating peptide (CPP); a cationic polymer or dendrimer e.g. polyethylenimine (PEI) and poly-D,L-lactide-co-glycolide (PLGA); and/or a cationic lipid (e.g. lipofectamine). Preferably, the carrier is not a cationic agent. Preferably the carrier is not a cationic cell penetrating peptide (CPP); a cationic polymer or dendrimer e.g. polyethylenimine (PEI) and poly-D,L-lactide-co-glycolide (PLGA); and/or a cationic lipid (e.g. lipofectamine).

The carrier may be a small molecule (e.g., cholesterol, bile acid, and/or lipid), polymer, protein (e.g. an antibody), and/or aptamer (e.g. RNA) that is conjugated to the double-stranded siRNA molecule. The carrier may be a nanoparticulate formulation used to encapsulate the double-stranded siRNA molecule

The carrier may be a modification of the double-stranded siRNA molecule with a targeting ligand (e.g. an antibody), a peptide, a small molecule (e.g. folic acid and/or biotin), or a polymer present in extracellular matrix (e.g. hyaluronic acid and/or chondroitin sulphate), or a hydrophobic modification of the double-stranded siRNA molecule (e.g. using cholesterol and/or α-tocopherol). Preferably, the carrier is not a hydrophobic modification of the double-stranded siRNA molecule (e.g. using cholesterol and/or α-tocopherol).

The antisense strand of the double-stranded siRNA may have a 3'-overhang that is shorter than said 3'overhang of said sense strand. The double-stranded siRNA molecule may be any double-stranded siRNA molecule of the invention.

The disclosure further provides a cell obtainable by the methods of the invention. Thus, the cell may comprise a double-stranded siRNA molecule as described herein.

The disclosure further provides a method of target-specific ribonucleic acid (RNA) interference in a cell comprising contacting (in vitro) said cell with a double-stranded siRNA molecule, wherein the double-stranded siRNA molecule comprises a sense RNA strand and an antisense RNA strand, wherein the sense strand has a 3'-overhang of from four to eight nucleotides, and wherein at least at least a portion of the antisense strand of said double stranded siRNA molecule has a nucleotide sequence that is complementary to a nucleotide sequence of a target RNA sequence.

The step of contacting (in vitro) the cell with a double-stranded siRNA molecule may be performed in the absence of an agent for facilitating release of said siRNA from the endosome into the cytosol of said cell. The step of contacting (in vitro) the cell with a double-stranded siRNA molecule may be performed in the absence of an agent for facilitating entry of the double-stranded siRNA molecule into the cell. The step of contacting (in vitro) the cell with a double-stranded siRNA molecule may be performed in the absence of a cationic agent. The step of contacting (in vitro) the cell with a double-stranded siRNA molecule may be performed in the absence of a cationic cell penetrating peptide (CPP); a cationic polymer or dendrimer e.g. polyethylenimine (PEI) or poly-D,L-lactide-co-glycolide (PLGA); and/or a cationic lipid (e.g. lipofectamine).

The sequence of the antisense strand of the double-stranded siRNA molecule is selected, in the double-stranded portion or at least a portion thereof, to be complementary to and capable of hybridizing to a target RNA or DNA sequence, preferably a target mRNA sequence. The sequence of the sense strand is selected to be complementary to the antisense strand and form base pairs between the nucleotides in the respective strands in the double-stranded portion of the double-stranded siRNA molecule.

The sequence of the double-stranded siRNA molecule preferably has a sufficient identity to a target nucleotide sequence in order to mediate target-specific RNAi. Preferably, the sequence of the double-stranded portion has an identity to the desired target nucleotide sequence of at least 50%, at least 70%, at least 85 %, at least 90 %, at least 95 %, at least 96%, at least 97%, at least 98%, at least 99%, and most preferably 100 %. Preferably, the sequence of the double-stranded portion has identity to the desired target nucleotide sequence over at least 10, 11, 12, 13, 14, 15, 16, 17, 18, 19, 20, 21, 22, 23, 24, 25, 26, 27, 28, 29 or 30 (contiguous) nucleotides.

The cell may be contacted with the double-stranded siRNA molecule in the presence or absence of a carrier e.g. an agent or a formulation. Preferably, the carrier promotes accumulation of the double-stranded siRNA molecule at a target site and/or protects the double-stranded siRNA molecule from undesirable interactions with biological milieu components and/or protects the double-stranded siRNA molecule from metabolism and/or degradation.

The carrier may be a viral carrier or a non-viral carrier. Viral carriers include a lentiviral vector or an adenoviral vector for delivery of a DNA-based construct encoding the double-stranded siRNA molecule. Non-viral siRNA carriers (or vectors) include complexing the double-stranded siRNA molecule with a cationic agent such as a cationic cell penetrating peptide (CPP); a cationic polymer or dendrimer e.g. polyethylenimine (PEI) and poly-D,L-lactide-co-glycolide (PLGA); and/or a cationic lipid (e.g. lipofectamine). Preferably, the carrier is not a cationic agent. Preferably the carrier is not a cationic cell penetrating peptide (CPP); a cationic polymer or dendrimer e.g. polyethylenimine (PEI) and poly-D,L-lactide-co-glycolide (PLGA); and/or a cationic lipid (e.g. lipofectamine).

The carrier may be a small molecule (e.g., cholesterol, bile acid, and/or lipid), polymer, protein (e.g. an antibody), and/or aptamer (e.g. RNA) that is conjugated to the double-stranded siRNA molecule. The carrier may be a nanoparticulate formulation used to encapsulate the double-stranded siRNA molecule.

The carrier may be a modification of the double-stranded siRNA molecule with a targeting ligand (e.g. an antibody), a peptide, a small molecule (e.g. folic acid and/or biotin), or a polymer present in extracellular matrix (e.g. hyaluronic acid and/or chondroitin sulphate), or a hydrophobic modification of the double-stranded siRNA molecule (e.g. using cholesterol and/or α-tocopherol). Preferably, the carrier is not a hydrophobic modification of the double-stranded siRNA molecule (e.g. using cholesterol and/or α-tocopherol).

The antisense strand of the double-stranded siRNA molecule may have a 3'-overhang that is shorter than said 3'overhang of said sense strand. The double-stranded siRNA molecule may be any double-stranded siRNA molecule of the invention.

The disclosure further provides the use of a double-stranded small interfering ribonucleic acid (siRNA) molecule for target-specific ribonucleic acid (RNA) interference in a cell (in vitro), wherein the double-stranded siRNA molecule comprises a sense RNA strand and an antisense RNA strand, wherein the sense strand has a 3'-overhang of from four to eight nucleotides, and wherein the 3'-overhang facilitates release of the siRNA from the endosome into the cytosol of said cell.

The use of a double-stranded siRNA molecule for target-specific ribonucleic acid (RNA) interference in a cell (in vitro) may be in the absence of an agent for facilitating release of said siRNA from the endosome into the cytosol of said cell. The use of a double-stranded siRNA molecule for target-specific ribonucleic acid (RNA) interference in a cell (in vitro) may be in the absence of an agent for facilitating entry of the siRNA into the cell. The use of a double-stranded siRNA molecule for target-specific ribonucleic acid (RNA) interference in a cell (in vitro) may be in the absence of a cationic agent. The use of a double-stranded siRNA molecule for target-specific ribonucleic acid (RNA) interference in a cell (in vitro) may be in the absence of a cationic cell penetrating peptide (CPP); a cationic polymer or dendrimer e.g. polyethylenimine (PEI) or poly-D,L-lactide-co-glycolide (PLGA); and/or a cationic lipid (e.g. lipofectamine).

In order to mediate target-specific ribonucleic acid (RNA) interference in a cell at least at least a portion of the antisense strand of the double stranded siRNA molecule has a nucleotide sequence that is complementary to a nucleotide sequence of a target RNA sequence.

The sequence of the antisense strand is selected, in the double-stranded portion or at least a portion thereof, to be complementary to and capable of hybridizing to a target RNA or DNA sequence, preferably a target mRNA sequence. The sequence of the sense strand is selected to be complementary to the antisense strand and form base pairs between the nucleotides in the respective strands in the double-stranded portion of the double-stranded siRNA molecule.

The sequence of the double-stranded siRNA molecule preferably has a sufficient identity to a target nucleotide sequence in order to mediate target-specific RNAi. Preferably, the sequence of the double-stranded portion has an identity to the desired target nucleotide sequence of at least 50%, at least 70%, at least 85 %, at least 90 %, at least 95 %, at least 96%, at least 97%, at least 98%, at least 99%, and most preferably 100%. Preferably, the sequence of the double-stranded portion has identity to the desired target nucleotide sequence over at least 10, 11, 12, 13, 14, 15, 16, 17, 18, 19, 20, 21, 22, 23, 24, 25, 26, 27, 28, 29 or 30 (contiguous) nucleotides.

The double-stranded siRNA molecule may be used with or without a carrier e.g. an agent or a formulation. Preferably, the carrier promotes accumulation of the double-stranded siRNA molecule at a target site and/or protects the double-stranded siRNA molecule from undesirable interactions with biological milieu components and/or protects the double-stranded siRNA molecule from metabolism and/or degradation.

The carrier may be a viral carrier or a non-viral carrier. Viral carriers include a lentiviral vector or an adenoviral vector for delivery of a DNA-based construct encoding the double-stranded siRNA molecule. Non-viral siRNA carriers (or vectors) include complexing the double-stranded siRNA molecule with a cationic agent such as a cationic cell penetrating peptide (CPP); a cationic polymer or dendrimer e.g. polyethylenimine (PEI) and poly-D,L-lactide-co-glycolide (PLGA); and/or a cationic lipid (e.g. lipofectamine). Preferably, the carrier is not a cationic agent. Preferably the carrier is not a cationic cell penetrating peptide (CPP); a cationic polymer or dendrimer e.g. polyethylenimine (PEI) and poly-D,L-lactide-co-glycolide (PLGA); and/or a cationic lipid (e.g. lipofectamine).

The carrier may be a small molecule (e.g., cholesterol, bile acid, and/or lipid), polymer, protein (e.g. an antibody), and/or aptamer (e.g. RNA) that is conjugated to the double-stranded siRNA molecule. The carrier may be a nanoparticulate formulation used to encapsulate the double-stranded siRNA molecule.

The carrier may be a modification of the double-stranded siRNA molecule with a targeting ligand (e.g. an antibody), a peptide, a small molecule (e.g. folic acid and/or biotin), or a polymer present in extracellular matrix (e.g. hyaluronic acid and/or chondroitin sulphate), or a hydrophobic modification of the double-stranded siRNA molecule (e.g. using cholesterol and/or α-tocopherol). Preferably, the carrier is not a hydrophobic modification of the double-stranded siRNA molecule (e.g. using cholesterol and/or α-tocopherol).

The antisense strand of the double-stranded siRNA molecule may have a 3'-overhang that is shorter than said 3'overhang of said sense strand. The double-stranded siRNA molecule may be any double-stranded siRNA molecule of the invention.

The disclosure further provides a double-stranded siRNA molecule for use in the treatment of a disease by sequence-specific knockdown of a target RNA sequence, wherein the double-stranded siRNA molecule comprises a sense RNA strand and an antisense RNA strand, wherein the sense strand has a 3'-overhang of from four to eight nucleotides, wherein at least a portion of the antisense strand of the double-stranded siRNA molecule has a nucleotide sequence that is complementary to a nucleotide sequence of the target RNA sequence.

The double-stranded siRNA molecule may be administered to the subject in the absence of an agent for facilitating release of said siRNA from the endosome into the cytosol of a cell of the subject. The double-stranded siRNA molecule may be administered to the subject in the absence of an agent for facilitating entry of the siRNA into a cell of the subject. The double-stranded siRNA molecule may be administered to the subject in the absence of a cationic agent. The double-stranded siRNA molecule may be administered to the subject in the absence of a cationic cell penetrating peptide (CPP); a cationic polymer or dendrimer e.g. polyethylenimine (PEI) or poly-D,L-lactide-co-glycolide (PLGA); and/or a cationic lipid (e.g. lipofectamine).

The sequence of the antisense strand is selected, in the double-stranded portion or at least a portion thereof, to be complementary to and capable of hybridizing to a target RNA or DNA sequence, preferably a target mRNA sequence. The sequence of the sense strand is selected to be complementary to the antisense strand and form base pairs between the nucleotides in the respective strands in the double-stranded portion of the double-stranded siRNA molecule.

The sequence of the double-stranded siRNA molecule preferably has a sufficient identity to a target nucleotide sequence in order to mediate target-specific RNAi. Preferably, the sequence of the double-stranded portion has an identity to the desired target nucleotide sequence of at least 50%, at least 70%, at least 85 %, at least 90 %, at least 95 %, at least 96%, at least 97%, at least 98%, at least 99%, and most preferably 100%. Preferably, the sequence of the double-stranded portion has identity to the desired target nucleotide sequence over at least 10, 11, 12, 13, 14, 15, 16, 17, 18, 19, 20, 21, 22, 23, 24, 25, 26, 27, 28, 29 or 30 (contiguous) nucleotides.

The double-stranded siRNA molecule may be administered with or without a carrier e.g. an agent or a formulation. Preferably, the carrier promotes accumulation of the double-stranded siRNA molecule at a target site and/or protects the double-stranded siRNA molecule from undesirable interactions with biological milieu components and/or protects the double-stranded siRNA molecule from metabolism and/or degradation.

The carrier may be a viral carrier or a non-viral carrier. Viral carriers include a lentiviral vector or an adenoviral vector for delivery of a DNA-based construct encoding the double-stranded siRNA molecule. Non-viral siRNA carriers (or vectors) include complexing the double-stranded siRNA molecule with a cationic agent such as a cationic cell penetrating peptide (CPP); a cationic polymer or dendrimer e.g. polyethylenimine (PEI) and poly-D,L-lactide-co-glycolide (PLGA); and/or a cationic lipid (e.g. lipofectamine). Preferably, the carrier is not a cationic agent. Preferably the carrier is not a cationic cell penetrating peptide (CPP); a cationic polymer or dendrimer e.g. polyethylenimine (PEI) and poly-D,L-lactide-co-glycolide (PLGA); and/or a cationic lipid (e.g. lipofectamine).

The carrier may be a small molecule (e.g., cholesterol, bile acid, and/or lipid), polymer, protein (e.g. an antibody), and/or aptamer (e.g. RNA) that is conjugated to the double-stranded siRNA molecule. The carrier may be a nanoparticulate formulation used to encapsulate the double-stranded siRNA molecule.

The carrier may be a modification of the double-stranded siRNA molecule with a targeting ligand (e.g. an antibody), a peptide, a small molecule (e.g. folic acid and/or biotin), or a polymer present in extracellular matrix (e.g. hyaluronic acid and/or chondroitin sulphate), or a hydrophobic modification of the double-stranded siRNA molecule (e.g. using cholesterol and/or α-tocopherol). Preferably, the carrier is not a hydrophobic modification of the double-stranded siRNA molecule (e.g. using cholesterol and/or α-tocopherol).

The antisense strand of the double-stranded siRNA may have a 3'-overhang that is shorter than the 3'overhang of said sense strand. The double-stranded siRNA molecule may be any double-stranded siRNA molecule of the invention.

The double-stranded siRNA molecule may be used to treat one or more of the diseases and/or disorders selected from genetic disorders, cancer (e.g. by silencing genes differentially upregulated in tumour cells and/or genes involved in cell division), HIV, other viral infections (e.g. infection caused by hepatitis A, hepatitis B, herpes simplex virus type 2, influenza, measles and/or respiratory syncytial virus), neurodegenerative diseases (e.g. Parkinson's disease and/or polyglutamine diseases such as Huntington's disease), ocular diseases (e.g. macular degeneration) and liver failure.

Potential antiviral therapies using the double-stranded siRNA molecule include one or more of the following: topical microbicide treatment to treat infection by herpes simplex virus type 2, inhibition of viral gene expression in cancerous cells, knockdown of host receptors and/or co-receptors for HIV, silencing of hepatitis A and/or hepatitis B genes, silencing of influenza gene expression, and inhibition of measles viral replication. Potential treatments for neurodegenerative diseases include treatment of polyglutamine diseases such as Huntington's disease.

A subject treated with the double-stranded siRNA molecule may receive the double-stranded siRNA molecule in combination with other forms of treatment for the disorder concerned, including treatment with drugs generally used for the treatment of the disorder. The drugs may be administered in one or several dosage units.

The disclosure further provides a method of treating a patient suffering from a disease comprising administering a double-stranded siRNA molecule to the patient, wherein the double-stranded siRNA molecule provides sequence-specific knockdown of a target RNA sequence in the patient, wherein said double-stranded siRNA molecule comprises a sense RNA strand and an antisense RNA strand, wherein the sense strand has a 3'-overhang of from four to eight nucleotides, and wherein at least a portion of an antisense strand of said double-stranded siRNA molecule has a nucleotide sequence that is complementary to a nucleotide sequence of said target RNA sequence.

The double-stranded siRNA molecule may be administered to the patient in the absence of an agent for facilitating release of the siRNA from the endosome into the cytosol of a cell of said patient. The double-stranded siRNA molecule may be administered to the patient in the absence of an agent for facilitating entry of the siRNA into a cell of the patient. The double-stranded siRNA molecule may be administered to the patient in the absence of a cationic agent. The double-stranded siRNA molecule may be administered to the patient in the absence of a cationic cell penetrating peptide (CPP); a cationic polymer or dendrimer e.g. polyethylenimine (PEI) or poly-D,L-lactide-co-glycolide (PLGA); and/or a cationic lipid (e.g. lipofectamine).

The sequence of the antisense strand is selected, in the double-stranded portion or at least a portion thereof, to be complementary to and capable of hybridizing to a target RNA or DNA sequence, preferably a target mRNA sequence. The sequence of the sense strand is selected to be complementary to the antisense strand and form base pairs between the nucleotides in the respective strands in the double-stranded portion of the double-stranded siRNA molecule.

The sequence of the double-stranded siRNA molecule preferably has a sufficient identity to a target nucleotide sequence in order to mediate target-specific RNAi. Preferably, the sequence of the double-stranded portion has an identity to the desired target nucleotide sequence of at least 50%, at least 70%, at least 85 %, at least 90 %, at least 95 %, at least 96%, at least 97%, at least 98%, at least 99%, and most preferably 100%. Preferably, the sequence of the double-stranded portion has identity to the desired target nucleotide sequence over at least 10, 11, 12, 13, 14, 15, 16, 17, 18, 19, 20, 21, 22, 23, 24, 25, 26, 27, 28, 29 or 30 (contiguous) nucleotides.

The double-stranded siRNA molecule may be administered to the patient with or without a carrier e.g. an agent or a formulation. Preferably, the carrier promotes accumulation of the double-stranded siRNA molecule at a target site and/or protects the double-stranded siRNA molecule from undesirable interactions with biological milieu components and/or protects the double-stranded siRNA molecule from metabolism and/or degradation.

The carrier may be a viral carrier or a non-viral carrier. Viral carriers include a lentiviral vector or an adenoviral vector for delivery of a DNA-based construct encoding the double-stranded siRNA molecule. Non-viral siRNA carriers (or vectors) include complexing the double-stranded siRNA molecule with a cationic agent such as a cationic cell penetrating peptide (CPP); a cationic polymer or dendrimer e.g. polyethylenimine (PEI) and poly-D,L-lactide-co-glycolide (PLGA); and/or a cationic lipid (e.g. lipofectamine). Preferably, the carrier is not a cationic agent. Preferably the carrier is not a cationic cell penetrating peptide (CPP); a cationic polymer or dendrimer e.g. polyethylenimine (PEI) and poly-D,L-lactide-co-glycolide (PLGA); and/or a cationic lipid (e.g. lipofectamine).

The carrier may be a small molecule (e.g., cholesterol, bile acid, and/or lipid), polymer, protein (e.g. an antibody), and/or aptamer (e.g. RNA) that is conjugated to the double-stranded siRNA molecule. The carrier may be a nanoparticulate formulation used to encapsulate the double-stranded siRNA molecule.

The carrier may be a modification of the double-stranded siRNA molecule with a targeting ligand (e.g. an antibody), a peptide, a small molecule (e.g. folic acid and/or biotin), or a polymer present in extracellular matrix (e.g. hyaluronic acid and/or chondroitin sulphate), or a hydrophobic modification of the double-stranded siRNA molecule (e.g. using cholesterol and/or α-tocopherol). Preferably, the carrier is not a hydrophobic modification of the double-stranded siRNA molecule (e.g. using cholesterol and/or α-tocopherol).
The antisense strand of the double-stranded siRNA molecule may have a 3'-overhang that is shorter than said 3'overhang of said sense strand. The double-stranded siRNA molecule may be any double-stranded siRNA molecule of the invention.
The double-stranded siRNA molecule may be used to treat one or more of the diseases and/or disorders selected from genetic disorders, cancer (e.g. by silencing genes differentially upregulated in tumour cells and/or genes involved in cell division), HIV, other viral infections (e.g. infection caused by hepatitis A, hepatitis B, herpes simplex virus type 2, influenza, measles and/or respiratory syncytial virus), neurodegenerative diseases (e.g. Parkinson's disease and/or polyglutamine diseases such as Huntington's disease), ocular diseases (e.g. macular degeneration) and liver failure.
Potential antiviral therapies using the double-stranded siRNA molecule include one or more of the following: topical microbicide treatment to treat infection by herpes simplex virus type 2, inhibition of viral gene expression in cancerous cells, knockdown of host receptors and/or co-receptors for HIV, silencing of hepatitis A and/or hepatitis B genes, silencing of influenza gene expression, and inhibition of measles viral replication. Potential treatments for neurodegenerative diseases include treatment of polyglutamine diseases such as Huntington's disease.
A subject treated with the double-stranded siRNA molecule may receive the double-stranded siRNA molecule in combination with other forms of treatment for the disorder concerned, including treatment with drugs generally used for the treatment of the disorder. The drugs may be administered in one or several dosage units.

The disclosure further provides a double-stranded siRNA molecule comprising a sense RNA strand and an antisense RNA strand. The sense strand has a 3'-overhang of from four to eight nucleotides, of which at least one nucleotide is a deoxynucleotide.

The disclosure further provides a cell transfection method comprising contacting a cell to be transfected with a double-stranded siRNA molecule according to above.

The disclosure further provides a method of target-specific RNA interference in a cell. The method comprises contacting the cell with a double-stranded siRNA molecule according to above. At least a portion of an antisense strand of the double-stranded siRNA molecule has a nucleotide sequence that is complementary to a nucleotide sequence of a target RNA sequence.
The invention further provides a double-stranded siRNA molecule of the invention for use as a medicament and a double-stranded siRNA molecule of the invention for use in treating a disease by sequence-specific knockdown of a target RNA sequence. At least a portion of an antisense strand of the double-stranded siRNA molecule has a nucleotide sequence that is complementary to a nucleotide sequence of the target RNA sequence.
The invention further provides a pharmaceutical composition comprising a double-stranded siRNA molecule of the invention and a pharmaceutically acceptable diluent.

The disclosure further provides a cell transfected with a double-stranded siRNA molecule of the invention. The disclosure provides a nucleotide sequence encoding the double-stranded siRNA molecule.
The disclosure further provides a cell transfection method comprising contacting, in the absence of any transfection agent, a cell to be transfected with a double-stranded siRNA molecule comprising a sense RNA strand and an antisense RNA strand. The sense strand has a 3'-overhang of at least four nucleotides.
The double-stranded siRNA molecules of the embodiments have improved characteristics in terms of cellular uptake, endosomal escape and increase gene knockdown activity.

The double-stranded siRNA molecules of the invention mediate sequence-specific knockdown of a target RNA sequence. The % knockdown of the target RNA sequence may be at least 20%, at least 30%, at least 40%, at least 50%, at least 60%, at least 70%, at least 75%, at least 80%, at least 85%, at least 90%, or at least 95%, when compared to the normal level of expression of the target RNA sequence. The % knockdown of the target RNA sequence by a double-stranded siRNA molecule of the invention may be at least 1.5 times, at least 1.75 times, at least 2 times, at least 2.25 times, at least 2.5 times, at least 2.75 times, at least 3 times, at least 3.25 times, at least 3.5 times, at least 3.75 times, or at least 4 times the level of the knockdown achieved by a canonical siRNA i.e. an siRNA molecule having a double-stranded region identical to the double-stranded region of the siRNA of the invention with a sense strand 3' overhang of 2 nucleotides and an antisense strand 3' overhang of 2 nucleotides (e.g. a sense strand 3' overhang of dT₂ and an antisense strand 3' overhang of dT₂). The % knockdown of a target RNA may be determined by Real-Time PCR (RT-PCR).

The disclosure further provides a kit comprising a double-stranded siRNA molecule or composition of the invention. The kit may be suitable or intended for performing a method of the invention. The kit may further comprise one or more additional agents or reagents for performing one or more of the steps of the methods of the invention.

### BRIEF DESCRIPTION OF THE DRAWINGS

The embodiments, together with further objects and advantages thereof, may best be understood by making reference to the following description taken together with the accompanying drawings, in which:
Figs. 1A-1C illustrate various embodiments of double-stranded siRNA molecules.
Fig. 2A is a diagram showing GAPDH gene knockdown in MG63 cells transfected with cpRNA having different lengths of 3'-overhangs, namely, dT₂, dT₅, dT₈, as(dT₅), bl(dT₅) and (dT₅)₂, with or without CQ or MATra-si (M). The percentage of GAPDH knockdown was analyzed by RT-PCR (*P<0.005).
Fig. 2B is a diagram showing GAPDH gene knockdown in MG63 cells with different cpRNA sequences with or without CQ.
Fig. 3A is a diagram showing carrier-free transfection experiments using siRNA dT₂, and cpRNAs (dT₅ and dA₅) in MG63, HOB, HCT116, primary human keratinocytes and primary human fibroblast cells. The percentage of GAPDH knockdown was determined using RT-PCR experiments (***P<0.0001).
Fig. 3B illustrates a time-course plot showing uptake kinetics of Cy3-cpRNA (50 nM) as determined from flow cytometry using MG63 cells in presence or absence of 600 nM ODN2006 (pre-incubated for 1 h).
Fig. 3C illustrates fluorescence assisted cell sorting (FACS) histogram displaying uptake of Cy3-cpRNA (50 nM) in MG63, HOB, HCT116, HEK293, and MC3T3 cells after 24 h of incubation. Untreated control cells are shown in grey.
Fig. 4 is a diagram showing that cellular uptake of cpRNA is an energy dependent process. HCT116, HOB and MG63 cells were transfected with dT₅ and dA₅ a 4 °C or 37 °C. Cells were also transfected with negative control siRNA and cells left untreated were taken as controls and the percentage of GAPDH knockdown was analyzed by RT-PCR (***P<0.0001).
Fig. 5 illustrates cell proliferation assessed by MTS assay, 24 h post transfection. MG63 cells were transfected with siRNA sequences having different overhang lengths and negative control (NC) siRNAs at 50 nM and 100 nM, respectively, with (wM) or without MATra-si (woM). Control (C) wells were left untreated, and cell viability in control cells was defined as 1.
Fig. 6A-6C illustrates immunostimulatory effects of cpRNA. The expression level of IFN-α (Fig. 6A), IFN-β (Fig. 6B) and IFN-γ (Fig. 6C) mRNA was measured by RT-PCR at 24 h post-transfection with 50 or 100 nM of siRNA having different overhang lengths and negative control (NC), as indicated in the figures. Transfection experiments were performed in MG63 cells with (wM) or without MATra-si (woM). Expression levels were normalized to that of β-actin. The relative expression of IFN was defined as 1 in control (C) cells.
Fig. 7 (A) shows dose dependent knockdown of normal siRNA (dT₂) and siRNA (dT₅) (cpRNA). The concentration of 50 nM cpRNA displayed the highest knockdown of 80%, which did not increase with higher concentration; (B) Confocal images of MG63 cells treated with normal siRNA (dT₂) showing with localized distribution to endosome; and (C) using cpRNA (dT₅) which displayed perinuclear localization within cytosol.
Fig. 8 Durable and efficacious gene silencing induced by cpRNA (dT₅) in MG63 cells. Cells were transfected with scrambled siRNA (dT₅) (C), canonical siRNA (dT₂) with or without MATra (M), and cpRNA (dT₅) targeting GAPDH and CTNNB1 at the concentration of 50nM. After 24, 48 and 72 h, mRNA expression of (A) GAPDH and (B) CTNNB1 was measured using RT-PCR, and the protein levels were further analysed using Western blot; β-actin was used as the loading control.
Fig. 9 illustrates GFP knockdown experiments. Fig. 9A control GFP expressing MG63 cells. Fig. 9B GFP expressing MG63 cells treated with 50 nM GFP-siRNA. Fig. 9C GFP expressing MG63 cells treated with 50 nM GFP-cpRNA.

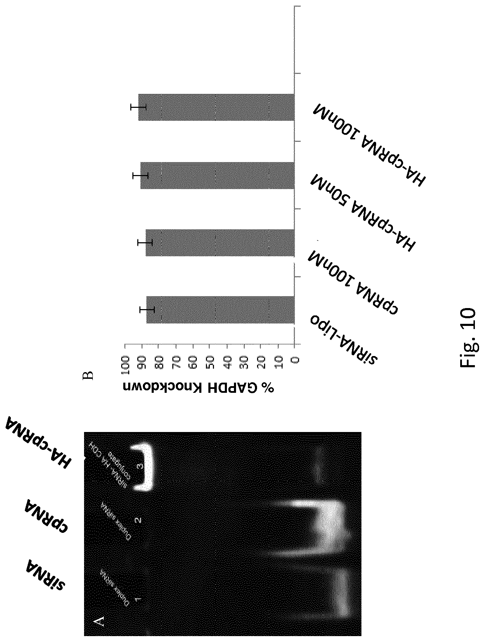

Fig. 10 illustrates the conjugation of aldehyde modified siRNA with hydrazide modified hyaluronan or HA (A) Gel electrophoresis assay confirming the siRNA aldehyde-HA hydrazide conjugate. The first lane corresponds to normal siRNA dT₂ (siRNA); the second lane corresponds to aldehyde-modified GAPDH siRNA (dT₅) (cpRNA); the third lane shows efficient conjugation of aldehyde-modified GAPDH siRNA (dT₅) to HA by hydrazone linkage (HA-cpRNA). (B) qPCR analysis showing efficient gene knockdown upon conjugation with HA. Normal siRNA (dT₂) with transfection reagent (lipofectamine) is shown in first bar (siRNA-Lipo), followed by cpRNA (dT₅) and HA-cpRNA (dT₅) conjugates at 50 and 100 nM concentrations.

### DETAILED DESCRIPTION

The present disclosure relates to double-stranded small interfering ribonucleic acid (siRNA) molecules possessing a small DNA/RNA sequence (overhang) at the 3'-end of the sense strand, also referred to as the passenger strand. The double-stranded siRNA of the present embodiments display unique carrier-free cellular uptake, endosomal escape and increased knockdown activity against target gene of interest. As a consequence, the double-stranded siRNA molecule is also referred to as cell penetrating siRNA or cpRNA herein.

The present disclosure also relates to a method of production of a double-stranded siRNA molecule with 3'-overhang on the sense strand, a cell transfection method, a method of target-specific RNA interference (RNAi) and pharmaceutical compositions with the double-stranded siRNA molecules according to the present disclosure.

Hence, the disclosure provides a double-stranded siRNA molecule comprising a sense RNA strand and an antisense RNA strand. The sense strand of the siRNA molecule has a 3'-overhang of from four to eight nucleotides. In an instance, at least one of these four to eight nucleotides is a deoxynucleotide.

Thus, the present disclosure is based on the surprising finding that having a 3'-overhang of specific length and preferably comprising one or more deoxynucleotides at the sense or passenger strand enables the double-stranded siRNA molecule to be taken up by cells without the need of any added carrier or transfection agent. The double-stranded siRNA molecule with the particular 3'-overhang at the sense strand further has improved characteristics in terms of endosomal escape and increased knockdown activity as compared to prior art siRNA molecules.

The at least one deoxynucleotide of the 3'-overhang could be any deoxynucleotide or combination of at least two deoxynucleotides, such as deoxythymidine (dT), deoxyadenosine (dA), deoxyguanosine (dG) or deoxycytosine (dC).

In a particular embodiment, all of the four to eight nucleotides of the 3'-overhang of the sense strand are preferably deoxynucleotides. In such an approach, all deoxynucleotides could be dT, all could be dA, all could be dG or all could be dC. Alternatively, all deoxynucleotides could be purine deoxynucleotides, i.e. dG and/or dA, or all could be pyrimidine deoxynucleotides, i.e. dT and/or dC. In yet another approach, a mixture of purine and pyrimidine deoxynucleotides could be used, such as a mixture of dA, dG, dT and/or dC.

Non-limiting examples of 3-overhangs for the sense strand include dT₄, dA₄, dG₄, dC₄, dT₅, dA₅, dG₅, dC₅, dT₆, dA₆, dG₆, dC₆, dT₇, dA₇, dG₇, dC₇, dT₈, dA₈, dG₈ and dC₈, preferably dT₅, dA₅, dG₅, dC₅, dT₆, dA₆, dG₆, dC₆, dT₇, dA₇, dG₇, dC₇, dT₈, dA₈, dG₈ and dC₈, more preferably dT₅, dA₅, dG₅, dC₅, dT₆, dA₆, dG₆, dC₆, dT₇, dA₇, dG₇ and dC₇, such as dT₅, dA₅, dG₅, dC₅, dT₆, dA₆, dG₆ and dC₆, or dT₅, dA₅, dG₅ and dC₅.

Although it is currently preferred to only use deoxynucleotides in the 3'-overhang of the sense strand, a portion of the four to eight nucleotides could be ribonucleotides and/or modified nucleotides. The ribonucleotides include riboadenosine (rA), riboguanosine (rG), ribouracil (rU) and ribocytosine (rC). Modified nucleotides can have modifications on the ribose sugar, the phosphate backbone and/or nucleobase. Non-limiting examples of ribose modifications are analogues to modifications where 2'-OH is replaced by H, SH, SR, R, OR, Cl, Br, I, F, CN, NH₂, NHR, NR₂, guanidine, wherein R is an optionally substituted aryl group, C1-C6 alkyl, C2-C6-alkenyl or C2-C6 alkynyl group. Further nucleotide analogues and nucleotide modifications that can be used according to the embodiments are disclosed in paragraph [0017] of EP 1 407 044.

In a particular embodiment, the sense strand has a 3'-overhang of from five to eight nucleotides. Experimental data as presented herein show that a 3'-overhang of the sense strand with five or eight nucleotides have improved characteristics as compared to prior art 3'-overhangs that generally include fewer nucleotides, such as two nucleotides. The 3'-overhang preferably consists of five or eight deoxynucleotides.

A particular embodiment involves a sense strand of the double-stranded siRNA molecule having a 3'-overhang of five nucleotides. Non-limiting but preferred examples include a 3'-overhang with five deoxynucleotides, such as selected from a group consisting of dT₅, dA₅, dG₅, dC₅. Among the different 3'-overhangs with five deoxynucleotides, dT₅ and dA₅ were the best candidates as shown in the presented examples.

In an embodiment, also the antisense or guide strand of the double-stranded siRNA molecule has a 3'-overhang. In such a case, the 3'-overhang of the antisense strand preferably has at least two nucleotides. In a particular embodiment, the 3'-overhang of the antisense strand preferably has from two to five nucleotides. A currently preferred embodiment is to have a 3'-overhang of the antisense strand with two nucleotides.

In an embodiment, at least one nucleotide in the 3'-overhang of the antisense strand is a deoxynucleotide. In a particular embodiment, all of the at least two, preferably two to five and more preferably two, nucleotides in the 3'-overhang of the antisense strand are deoxynucleotides. In such a case, the 3'-overhang of the antisense strand could, for instance, be selected from a group consisting of dT₂, dA₂, dG₂, dC₂, dT₃, dA₃, dG₃, dC₃, dT₄, dA₄, dG₄, dC₄, dT₅, dA₅, dG₅ and dC₅ In a particular embodiment, the group consists of dT₂, dA₂, dG₂, dC₂, dT₅, dA₅, dG₅ and dC₅, or preferably consists of dT₂, dA₂, dG₂, dC₂. Also a combination of different dexonucleotides are possible within the 3'-overhang of the antisense strand.

Although deoxynucleotides are preferably included in the 3'-overhang of the antisense strand all or at least a portion of the nucleotides could be ribonucleotides and/or modified nucleotides as discussed in the foregoing for the sense strand.

In another embodiment, the antisense strand of the double-stranded siRNA molecule does not have any 3'-overhang but rather has a blunt end.

The double-stranded siRNA molecule according to the embodiments preferably has a double-stranded or duplex portion with a length of at least 19 base pairs. For instance, the double-stranded portion could be of from 19 to 30 base pairs, preferably from 19 to 24 base pairs, such as 19, 20, 21, 22, 23 or 24 base pairs.

If the double-stranded siRNA molecule has a double-stranded portion of 19 base pairs, the sense strand preferably has a total length of 23 to 27 nucleotides. The antisense strand could then have a total length of 19 nucleotides if having a blunt end or preferably from 21 to 24 nucleotides, such as 21 nucleotides, with a 3'-overhang of two to five, such as two, nucleotides.

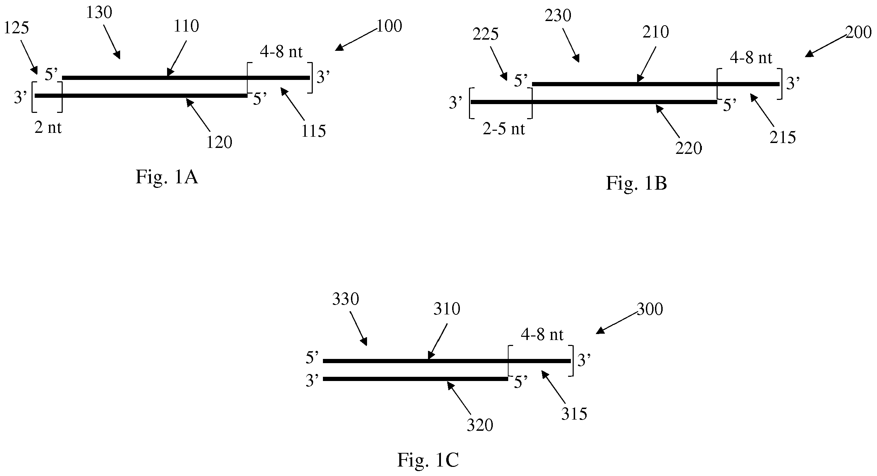

Figs. 1A-1C illustrates different embodiments of the double-stranded molecule 100, 200, 300. In Fig. 1A, the double-stranded molecule 100 has a sense strand 110 with a 3'-overhang 115 of four to eight nucleotides and an antisense strand 120 with a 3'-overhang 125 of two nucleotides. Fig. 1B illustrates a double-stranded siRNA molecule 200 where the sense strand 210 has a same four to eight nucleotide 3'-overhang 215 as in Fig. 1B, whereas the antisense strand 220 has a 3'-overhang of two to five nucleotides. Fig. 1C illustrates an embodiment of the double-stranded siRNA molecule 300, in which the antisense strand 320 has a blunt end. The sense strand 320 has a four to eight nucleotide long 3'-overhang 325.

In Figs. 1A-1C, reference numbers 130, 230, 330 denote the double-stranded portion of the double-stranded siRNA molecule 100, 200, 300.

This design of the double-stranded siRNA molecule according to the present embodiments can be applied to any double-stranded siRNA molecule. This means that the double-stranded siRNA molecule can have any nucleotide sequence in the double-stranded portion. Generally, the sequence of the antisense strand is selected, in the double-stranded portion or at least a portion thereof, to be complementary to and capable of hybridizing to a target RNA or DNA sequence, preferably a target mRNA sequence. The sequence of the sense strand is then selected to be complementary to the antisense strand and form base pairs between the nucleotides in the respective strands in the double-stranded portion of the double-stranded siRNA molecule.

The sequence of the double-stranded siRNA molecule preferably has a sufficient identity to a target nucleotide sequence in order to mediate target-specific RNAi. Preferably, the sequence has an identity of at least 50 %, particularly of at least 70 % to the desired target nucleotide sequence in the double-stranded portion of the double-stranded siRNA molecule. More preferably, the identity is at least 85 %, such as at least 90 % or 95 %, and most preferably 100 % in the double-stranded portion of the double-stranded siRNA molecule.

In further embodiments, the sense strand of the double-stranded siRNA molecule may contain labels at the 5'-end for detection in analytical, in particular diagnostic purpose. The 'label' can be any chemical entity which enable the detection of the double-stranded siRNA molecule via, physical, chemical and/or biological means. Examples of labels integrated on the 5'-end of the sense strand include chromophores, fluorophores or radioactive molecules.

The disclosure further provides a method of preparing a double-stranded siRNA molecule of the embodiments. The method generally comprises synthesizing a sense (RNA) strand having a 3'-overhang of from four to eight nucleotides, of which at least one nucleotide is a deoxynucleotide, and an antisense (RNA) strand. The sense strand and the antisense strand are capable of hybridizing in a double-stranded portion of the double-stranded siRNA molecule. The method preferably also comprises combing the sense strand and the antisense strand under conditions allowing hybridization for form the double-stranded siRNA molecule.

Methods of synthesizing RNA strands are well known in the art, including for instance phosphoramidite chemistry, H-phosphonate chemistry and enzymatic chain extension. The sense and antisense strands can also be prepared by enzymatic transcription from DNA templates.

The disclosure further provides a cell transfection method. The method comprises contacting a cell to be transfected with a double-stranded siRNA molecule according to the embodiments.

The double-stranded siRNA molecules of the embodiments have improved cell penetrating properties as compared to prior art siRNA molecules. Hence, the present double-stranded siRNA molecules generally do not need any transfection agent in order to transfect the cell and thereby introduce the double-stranded siRNA molecule into the cell. Hence, uptake of the double-stranded siRNA molecules of the embodiments by cells can take place without the need for various transfection agents.

Transfection agent as used herein includes various carriers traditionally employed within the area of RNAi when trying to introduce siRNA molecules into cells. Examples of such carriers include liposomal carriers; viruses as carriers, i.e. viral transduction; chemical carriers, such as using calcium phosphate to form a precipitate with the siRNA that can be taken up by the cell, highly branched organic carriers, denoted dendrimers, and cationic polymers, such as DEAE-dextran or polyethylenimine (PEI); nanoparticles; etc.

Transfection agent as used herein further includes various targeting molecules which could be conjugated or complexed with the siRNA and which could target specific receptors on the cell surface. Examples of such carriers include folic acid; biotin; antibody; biopolymers like hyaluronic acid; peptides; proteins; etc.

Transfection agent may further include techniques or methods to introduce the siRNA into the cell, including direction injection; electroporation; sonoporation; optical transfection; protoplast fusion; impalefection; hydrodynamic delivery; magnetofection; particle bombardment; and nucleofection.

Thus, cell transfection and gene knockdown using the double-stranded siRNA molecules of the embodiments does not require the use of any transfection agents. The double-stranded siRNA molecules according to the present embodiments can be administered and taken up by cells itself, in the absence of any delivery carrier, vector, hydrophobic modification, etc. of the double-stranded siRNA molecules.

Hence, in an embodiment, contacting the cell comprises contacting the cell to be transfected by the double-stranded siRNA molecule in the absence of a transfection agent.

In a particular embodiment, the cell or cells to be transfected may be housed *in vitro* in a cell culture medium in a Petri dish, culture vessel or well, etc. In such a case, the double-stranded siRNA molecules can simply be added to the cell culture medium or the cells could be added to a solution, such as saline, a buffered solution or a cell culture medium, comprising the double-stranded siRNA molecules.

Thus, in an embodiment contacting the cell comprises contacting *in vitro* the cell to be transfected by the double-stranded siRNA molecule in the absence of a transfection agent.

The double-stranded siRNA molecule may be administered to an organism in which RNAi and gene knockdown is desired. The organism could, for instance, be an animal, such as mammal, including a human, a fungus, a micro-organism, or a plant.

The double-stranded siRNA molecule can then preferably be provided as a composition comprising, in addition to the double-stranded siRNA molecule, a diluent, such as saline or a buffered solution, such as a buffered aqueous solution.

The disclosure further provides a method of target-specific RNAi in a cell. The method comprises contacting the cell with a double-stranded siRNA molecule according to the embodiments. In such a case, at least a portion of an antisense strand of the double-stranded siRNA molecule has a nucleotide sequence that is complementary to a nucleotide sequence of a target RNA sequence.

Hence, the antisense strand of the double-stranded siRNA molecule has a nucleotide sequence selected to enable the antisense strand to bind and hybridize to the target RNA sequence. As a consequence of this hybridization, the target RNA sequence will be bound to an activated RISC complex in a sequence-specific manner, resulting in RNAi.

The target RNA sequence is preferably a target mRNA sequence to thereby achieve inhibition or reduction, such as know-down, of gene expression with regard to the gene encoding the mRNA.

The method preferably comprises contacting the cell with the double-stranded siRNA molecule in the absence of a transfection agent.

In an instance, contacting the cell comprises contacting the cell *in vitro* with the double-stranded siRNA molecule.

In another instance, contacting the cell comprises administering the double-stranded siRNA molecule to an organism to achieve the target-specific RNAi in the organism.

The double-stranded siRNA molecule is preferably administered to the organism within a diluent and as a composition. The composition may be in form of a solution, e.g. an injectable solution, a cream, ointment, tablet, suspension or the like. The composition may be administered in any suitable way, e.g. by injection, by oral, topical, nasal, rectal application etc. The diluent may be any suitable pharmaceutically acceptable diluent.

The invention further provides a double-stranded siRNA molecule according to the invention for use as a medicament. The invention further provides a double-stranded siRNA molecule according to the invention for use in treating, inhibiting or preventing a disease by sequence-specific knockdown (RNAi) of a target RNA sequence. In such a case, at least a portion of an antisense stand of the double-stranded siRNA molecule has a nucleotide sequence that is complementary to a nucleotide sequence of the target RNA sequence.

The disease can be any disease, disorder or medical condition that can be treated, inhibited or prevented by RNAi and more particularly by prevent expression of a target gene in the organism. For example, the target gene may be a pathogen-associated gene, e.g. a viral gene, a tumor-associated gene or an autoimmune disease-associated gene. The target gene may also be a heterologous gene expressed in a recombinant cell or a genetically altered organism. By inhibiting the function of such a gene therapeutic benefits in the agricultural field or in the medicine or veterinary medicine field may be obtained.

Disorders in which treatment with the double-stranded siRNA molecule would be an option, including, for instance, genetic disorders, cancer, HIV, other viral infections, Parkinson, regenerative medicine, ocular diseases.

A subject treated with siRNA may receive the double-stranded siRNA molecule in combination with other forms of treatment of the disorder concerned, including treatment with drugs generally used for the treatment of the disorder. The drugs may be administered in one or several dosage units.
It is possible to exploit RNAi in therapy. Among the first applications to reach clinical trials were in the treatment of macular degeneration and respiratory syncytial virus. RNAi has also been shown to be effective in reversing induced liver failure.

Potential antiviral therapies include topical microbicide treatments that use RNAi to treat infection by herpes simplex virus type 2 and the inhibition of viral gene expression in cancerous cells, knockdown of host receptors and coreceptors for HIV, the silencing of hepatitis A and hepatitis B genes, silencing of influenza gene expression, and inhibition of measles viral replication. Potential treatments for neurodegenerative diseases have also been proposed, with particular attention to polyglutamine diseases such as Huntington's disease.
RNAi is also a promising way to treat cancers by silencing genes differentially upregulated in tumor cells or genes involved in cell division.
The RNAi pathway is often exploited in experimental biology to study the function of genes in cell culture and *in vivo* in model organisms. Double-stranded siRNA molecules are synthesized with a sequence complementary to a gene of interest and introduced into a cell or organism, where it is recognized as exogenous genetic material and activates the RNAi pathway. Using this mechanism, researchers can cause a drastic decrease in the expression of a targeted gene. Studying the effects of this decrease can show the physiological role of the gene product. Since RNAi may not totally abolish expression of the gene, this technique is sometimes referred as a "knockdown", to distinguish it from "knockout" procedures in which expression of a gene is entirely eliminated.
Most functional genomics applications of RNAi in animals have used *C*. *elegans* and *Drosophila*, as these are the common model organisms in which RNAi is most effective.
Approaches to the design of genome-wide RNAi libraries can require more sophistication than the design of a single siRNA for a defined set of experimental conditions. Artificial neural networks are frequently used to design siRNA libraries and to predict their likely efficiency at gene knockdown. Mass genomic screening is widely seen as a promising method for genome annotation and has triggered the development of high-throughput screening methods based on microarrays.

Functional genomics using RNAi is a particularly attractive technique for genomic mapping and annotation in plants because many plants are polyploid, which presents substantial challenges for more traditional genetic engineering methods. For example, RNAi has been successfully used for functional genomics studies in bread wheat (which is hexaploid) as well as more common plant model systems *Arabidopsis* and maize.

RNAi has been used for applications in biotechnology and is nearing commercialization in others.

RNAi has been used to genetically engineer plants to produce lower levels of natural plant toxins. Such techniques take advantage of the stable and heritable RNAi phenotype in plant stocks. Cotton seeds are rich in dietary protein but naturally contain the toxic terpenoid product gossypol, making them unsuitable for human consumption. RNAi has been used to produce cotton stocks whose seeds contain reduced levels of delta-cadinene synthase, a key enzyme in gossypol production, without affecting the enzyme's production in other parts of the plant, where gossypol is itself important in preventing damage from plant pests. Similar efforts have been directed toward the reduction of the cyanogenic natural product linamarin in cassava plants.

RNAi is under development as an insecticide, employing multiple approaches, including genetic engineering and topical application. Cells in the midgut of many larvae take up the molecules and help spread the signal throughout the insect's body.

Transgenic crops have been made to express small bits of RNA, carefully chosen to silence crucial genes in target pests. RNAs exist that affect only insects that have specific genetic sequences.

Alternatively dsRNA can be supplied without genetic engineering. One approach is to add them to irrigation water. The molecules are absorbed into the plants' vascular system and poison insects feeding on them. Another approach involves spraying RNA like a conventional pesticide. This would allow faster adaptation to resistance.

Genome-scale RNAi research relies on high-throughput screening (HTS) technology. RNAi HTS technology allows genome-wide loss-of-function screening and is broadly used in the identification of genes associated with specific phenotypes. This technology has been hailed as the second genomics wave, following the first genomics wave of gene expression microarray and single nucleotide polymorphism discovery platforms. One major advantage of genome-scale RNAi screening is its ability to simultaneously interrogate thousands of genes. With the ability generate a large amount of data per experiment, genome-scale RNAi screening has led to an explosion data generation rates.
The invention further provides a pharmaceutical composition comprising a double-stranded siRNA molecule according to the invention and a pharmaceutically acceptable diluent. Non-limiting examples of pharmaceutically acceptable diluents include saline, buffered solutions, including buffered aqueous solutions or other excipients.

The disclosure further provides a cell transfected with a double-stranded siRNA molecule according to the invention. The disclosure provides a nucleotide sequence encoding the double stranded siRNA molecule.
In the latter case, the nucleotide sequence may be provided as an expression cassette comprising a promoter operatively linked to the nucleotide sequence to enable expression of the antisense strand and the sense strand of the double-stranded siRNA molecule in the cell. The expression cassette could be in the form of naked DNA or be included in a vector, such as a plasmid.
The disclosure further provides a cell transfection method comprising contacting, in the absence of any transfection agent, a cell to be transfected with a double-stranded siRNA molecule comprising a sense RNA strand and an antisense RNA strand. In this embodiment, the sense strand has a 3'-overhang of at least four nucleotides.
In an embodiment, the 3'-overhang has at least five nucleotides, such as at least five ribonucleotides, at least five deoxynucleotides or a combination of ribo- and deoxynucletoides.
In another embodiment, the 3'-overhang has at least five nucleotides, such as at least five ribonucleotides, at least five deoxynucleotides or a combination of ribo- and deoxynucleotides where the 2'-hydroxy of ribonucleotides are modified/replaced with hydrophobic (alkyl) and hydrophilic (amine, thiol) groups that is well known in the field.
In another embodiment, the phosphate backbone of the 3'-overhangs of siRNA is modified with other backbone molecules known in the field such as phosphorthioates.

The above mentioned modification/replacement of 2'-hydroxy groups and/or phosphate backbone could be applied to other embodiments disclosed herein.

The disclosure may be further defined in the following set of numbered clauses:
1. A double-stranded small interfering ribonucleic acid, siRNA, molecule (100, 200, 300) comprising a sense RNA strand (110, 210, 310) and an antisense RNA strand (120, 220, 320), wherein said sense strand (110, 210, 310) has a 3'-overhang (115, 215, 315) of from four to eight nucleotides, of which at least one nucleotide is a deoxynucleotide.
2. The double-stranded siRNA molecule according to clause 1, wherein all of said four to eight nucleotides in said 3'-overhang are deoxynucleotides.
3. The double-stranded siRNA molecule according to clause 1 or 2, wherein said sense strand (110, 210, 310) has a 3'-overhang (115, 215, 315) of from five to eight nucleotides.
4. The double-stranded siRNA molecule according to clause 3, wherein said sense strand (110, 210, 310) has a 3'-overhang (115, 215, 315) of five nucleotides.
5. The double-stranded siRNA molecule according to clause 4, wherein said sense strand (110, 210, 310) has a 3'-overhang (115, 215, 315) selected from a group consisting of dT₅, dA₅, dG₅ or dC₅, wherein dT represents deoxythymidine, dA represents deoxyadenosine, dG represents deoxyguanosine and dC represents deoxycytosine.
6. The double-stranded siRNA molecule according to any of the clauses 1-5, wherein said antisense strand (120, 220) has a 3'-overhang (125, 225) at least two nucleotides.
7. The double-stranded siRNA molecule according to clause 6, wherein said antisense strand (120, 220) has a 3'-overhang (125, 225) of from two to five nucleotides.
8. The double-stranded siRNA molecule according to clause 6 or 7, wherein said antisense strand (120) has a 3'-overhang (125) of two nucleotides.
9. The double-stranded siRNA molecule according to any of the clauses 6-8, wherein at least one nucleotide in said 3'-overhang (125, 225) of said antisense strand (120, 220) is a deoxynucleotide.
10. The double-stranded siRNA molecule according to clause 9, wherein all of said at least two nucleotides in said 3'-overhang (125, 225) of said antisense strand (120, 220) are deoxynucleotides.
11. The double-stranded siRNA molecule according to clause 10, wherein said antisense strand (120, 220) has a 3'-overhang (125, 225) selected from a group consisting of dT₂, dA₂, dG₂, dC₂, dT₅, dA₅, dG₅ or dC₅, wherein dT represents deoxythymidine, dA represents deoxyadenosine, dG represents deoxyguanosine and dC represents deoxycytosine.
12. The double-stranded siRNA molecule according to any of the clauses 1 to 11, wherein a double-stranded portion (130, 230, 330) of said double-stranded siRNA molecule (100, 200, 300) has a length of at least 19 base pairs.
13. A cell transfection method comprising contacting a cell to be transfected with a double-stranded small interfering ribonucleic acid, siRNA, molecule (100, 200, 300) according to any of the clauses 1 to 12.
14. The cell transfection method according to clause 13, wherein contacting said cell comprises contacting said cell to be transfected by said double-stranded siRNA molecule (100, 200, 300) in the absence of a transfection agent.
15. The cell transfection method according to clause 13 or 14, wherein contacting said cell comprises contacting *in vitro* said cell to be transfected by said double-stranded siRNA molecule (100, 200, 300).
16. A method of target-specific ribonucleic acid, RNA, interference in a cell comprising contacting said cell with a double-stranded small interfering RNA, siRNA, molecule (100, 200, 300) according to any of the clauses 1 to 12, wherein at least a portion of an antisense strand (120, 220, 320) of said double-stranded siRNA molecule (100, 200, 300) has a nucleotide sequence that is complementary to a nucleotide sequence of a target RNA sequence.
17. A double-stranded small interfering ribonucleic acid, siRNA, molecule (100, 200, 300) according to any of the clauses 1 to 12 for use as a medicament.
18. A double-stranded small interfering ribonucleic acid, siRNA, molecule (100, 200, 300) according to any of the clauses 1 to 12 for use in treating a disease by sequence-specific knockdown of a target RNA sequence, wherein at least a portion of an antisense strand (120, 220, 320) of said double-stranded siRNA molecule (100, 200, 300) has a nucleotide sequence that is complementary to a nucleotide sequence of said target RNA sequence.
19. A pharmaceutical composition comprising a double-stranded small interfering ribonucleic acid, siRNA, molecule (100, 200, 300) according to any of the clauses 1 to 12 and a pharmaceutically acceptable diluent.
20. A cell transfected with a double-stranded small interfering ribonucleic acid, siRNA, molecule (100, 200, 300) according to any of the clauses 1 to 12 or a nucleotide sequence encoding said double-stranded siRNA molecule (100, 200, 300).
21. A cell transfection method comprising contacting, in the absence of any transfection agent, a cell to be transfected with a double-stranded small interfering ribonucleic acid, siRNA, molecule (100, 200, 300) comprising a sense RNA strand (110, 210, 310) and an antisense RNA strand (120, 220, 320), wherein said sense strand (110, 210, 310) has a 3'-overhang (115, 215, 315) of at least four nucleotides.

### EXAMPLES

### Materials and Methods

All siRNA sequences used in the present examples were high-performance liquid chromatography (HPLC) purified and purchased from Sigma-Aldrich, Sweden. The lyophilized duplexes were resuspended in RNase free water at 100 µM stock concentrations and used as it is.

The following sequences were used in the examples:
Canonical glyceraldehyde 3-phosphate dehydrogenase (GAPDH) siRNA sequences, abbreviated dT₂ herein:
Sense: 5'-CCG AGC CAC AUC GCU CAG A dTdT-3' (SEQ ID NO: 1)
Antisense: 5'-UCU GAG CGA UGU GGC UCG G dTdT-3' (SEQ ID NO: 2)

**Table 3 - GAPDH siRNA sequences**

| | | SEQ ID NO: | Abbreviation |
|---|---|---|---|
| sense 5'-3' | CCG AGC CAC AUC GCU CAG A **dT**₅ | 3 | dT₅ |
| antisense 5'-3' | UCU GAG CGA UGU GGC UCG G **dT**₂ | 2 | |
| sense 5'-3' | CCG AGC CAC AUC GCU CAG A **dT**₈ | 4 | dT₈ |
| antisense 5'-3' | UCU GAG CGA UGU GGC UCG G **dT**₂ | 2 | |
| sense 5'-3' | CCG AGC CAC AUC GCU CAG A **dT₅** | 3 | (dT₅)₂ |
| antisense 5'-3' | UCU GAG CGA UGU GGC UCG G **dT₅** | 5 | |
| sense 5'-3' | CCG AGC CAC AUC GCU CAG A **dA₅** | 6 | dA₅ |
| antisense 5'-3' | UCU GAG CGA UGU GGC UCG G **dT₂** | 2 | |
| sense 5'-3' | CCG AGC CAC AUC GCU CAG A **dG₅** | 7 | dG₅ |
| antisense 5'-3' | UCU GAG CGA UGU GGC UCG G **dT₂** | 2 | |
| sense 5'-3' | CCG *AGC CAC AUC GCU CAG A **dC₅** | 8 | dC₅ |
| antisense 5'-3' | UCU GAG CGA UGU GGC UCG G **dT₂** | 2 | |
| sense 5'-3' | CCG AGC CAC AUC GCU CAG A **A₅** | 9 | rA₅ |
| antisense 5'-3' | UCU GAG CGA UGU GGC UCG G **U₂** | 10 | |
| sense 5'-3' | CCG AGC CAC AUC GCU CAG A **dT₂** | 1 | as(dT₅) |
| antisense 5'-3' | UCU GAG CGA UGU GGC UCG G **dT**₅ | 5 | |
| sense 5'-3' | CCG AGC CAC AUC GCU CAG A **dT₅** | 3 | bl(dT₅) |
| antisense 5'-3' | UCU GAG CGA UGU GGC UCG G | 11 | |
| sense 5'-3' | Cy3-CCG AGC CAC AUC GCU CAG A **dT₅** | 12 | Cy3 cpRNA |
| antisense 5'-3' | UCU GAG CGA UGU GGC UCG G **dT₂** | 2 | |

*GFP*-siRNA sequences:

| | |
|---|---|
| Sense | 5'-GCAAGCUGACCCUGAAGUUdTdT-3' (SEQ ID NO: 13) |
| Antisense | 5'-AACUUCAGGGUCAGCUUGCdTdT-3' (SEQ ID NO: 14) |

*GFP*-cpRNA sequences:

| | |
|---|---|
| Sense | 5'-GCAAGCUGACCCUGAAGUUdTdTdTdTdT-3' (SEQ ID NO: 15) |
| Antisense | 5'-AACUUCAGGGUCAGCUUGCdTdT-3' (SEQ ID NO: 14) |

β-Catenin siRNA sequences

| | |
|---|---|
| Sense | 5'-GUA GCU GAU AUU GAU GGA C dTdT-3' (SEQ ID NO: 16) |
| Antisense | 5'-GUC CAU CAA UAU CAG CUA C dTdT-3' (SEQ ID NO: 17) |

β-Catenin cpRNA sequences

| | |
|---|---|
| Sense | 5'-GUA GCU GAU AUU GAU GGA C dTdTdTdTdT-3' (SEQ ID NO: 18) |
| Antisense | 5'-GUC CAU CAA UAU CAG CUA C dTdT-3' (SEQ ID NO: 17) |

Aldehyde-modified GAPDH cpRNA sequences

| | |
|---|---|
| Sense: | 5'-CCG AGC CAC AUC GCU CAG A dTdTdTdTdTU -3' (SEQ ID NO: 19) |
| Antisense: | 5'-UCU GAG CGA UGU GGC UCG G dTdT-3' (SEQ ID NO: 2) |

Negative control siRNAs (scrambled sequence) was: Stealth RNAi ™ siRNA Negative Control Lo GC (Invitrogen, part number: NC: 12935-200).

### Thermal melting studies

Thermal denaturation studies of siRNA was carried out in phosphate buffer, pH 7.0, containing 140 mM KCI. UV absorbance was monitored at 260 nm in the temperature range from 40 to 95 °C using a Lambda 35 UV-Vis spectrophotometer equipped with a Peltier temperature programmer with a heating rate of 0.5 °C/min. Samples (1 µM RNA) were denatured at 90 °C for 3 min followed by slow cooling to 27 °C prior to the measurements. Tm values were obtained from the maxima of the first derivatives of the melting curves. All Tm values given are the averages of three independent sets of experiments (±0.3 °C error range).

### Tuning thermodynamic asymmetry

Canonical siRNA (19 bp + 2 overhang) with the desired thermodynamic asymmetry is often identified using computational methods, which may not perfectly predict highly functional siRNA. These methods cannot be applied in certain cases, such as targeting point mutations or alternatively spliced isoforms with unique exons, because only a limited number of relevant siRNA could be obtained. Hence, siRNA sequences with the desired 5'-end thermodynamic asymmetry that would be applicable to any siRNA sequence without chemical modifications was sought. To achieve this aim, the overhang length of the 3'-end of the sense strand was extended from two nucleotides (nt) to five or eight. It was hypothesized that this strand extension ('wagging tail') at one end (3'-overhang in sense strand) of the siRNA duplex would induce thermodynamic destabilization, which could facilitate ATP-dependent selective unwinding and RISC recruitment of the desired strand.

The siRNA melting studies clearly revealed that increasing the overhang length using five dT repeats (dT₅) resulted in a drop in Tm of about 0.5 °C while an overhang with eight dTs (dT₈) resulted in a drop of 1.5 °C (as compared to canonical siRNA). Incorporating the dT₅ overhang at both ends (dT₅)₂ (i.e., extension of the 3'-end of the sense and antisense strands) resulted in a drop in Tm of ∼1 °C. These results clearly indicate symmetrical destabilization resulting in unwinding from both ends (corresponding to a Tm drop of 0.5 °C from each end).

By changing the nt type from pyrimidines to purines, a larger drop in Tm of ∼1.5 °C was observed with five dA (dA₅) overhangs and by ∼2 °C with five dG (dG₅) overhangs (see Table 3 for details of sequences). This change is presumably the result of the more ordered single-stranded structure of dA₅ and dG₅ as compared to dT₅. Other nt modifications were also tested, and their melting characteristics are shown in Table 4.

**Table 4 - Thermal denaturation of siRNA having different 3'overhang length**

| siRNA 3'-overhang | dT₂ | dT₅ | dT₈ | (dT₅)₂ | dA₅ | dG₅ | dC₅ | rA₅ | as(dT₅) | bl(dT₅) |
|---|---|---|---|---|---|---|---|---|---|---|
| *T*ₘ (°C) | 81.2 | 80.6 | 79.8 | 80.1 | 79.6 | 79.2 | 80.9 | 79.0 | 79.5 | 79.1 |
| Δ *T*ₘ (°C) | | -0.6 | -1.4 | -1.1 | -1.6 | -2.0 | -0.3 | -2.2 | -1.7 | -2.1 |

| | | | | | | | | | | |
|---|---|---|---|---|---|---|---|---|---|---|
| *Tₘ* values measured as the maximum of the first derivative of the melting curve (A₂₆₀ versus temperature) recorded in medium salt buffer (7.5 mM phosphate buffer pH 7.0 containing 140 mM KCl,) with a temperature range of 40-95 °C using 1 µM concentrations of the two complementary strands. Δ *Tₘ* = *Tₘ* relative to canonical siRNA (dT₂). | | | | | | | | | | |

### Cell culture and transfection

For all cell experiments, unless otherwise stated, cells were seeded at a density of 35 000 cells in a 24-well cell culture plate containing α-Minimum Essential Medium (α-MEM) (Sigma-Aldrich, Haverhill, UK) supplemented with 2 mM L-glutamine, 100 U/µL penicillin, 100 mg/µL streptomycin and 10 % fetal bovine serum (heat inactivated, Sigma-Aldrich) at 37 °C with 5 % CO₂ until confluence was reached.

Primary human osteoblast (HOB) cells isolated from human trabecular bone [1, 2] and keratinocytes and fibroblasts cells were isolated from skin tissue from healthy human donors. MG63 (human osteosarcoma cell line), HCT116 (human colon cancer cell line), HEK293 (human embryonic kidney 293 cells), C2C12 (mouse myoblast cell line) and MC3T3 (mouse osteoblast precursor cell line) cell lines were obtained from American Type Culture Collection, ATCC (Manassas, VA, USA). Cells were cultured until confluence was reached. The human cell experiments were approved by the local ethics committee (Ethical approval # Ups 03-561).

At 70 % confluency, a day prior to transfection, the cells were seeded at a density of 35 000 cells in a 24-well cell culture plate to achieve 60-80 % confluence at the day of transfection. Cell penetrating siRNAs (cpRNAs) of different lengths were transfected at 50 nM concentrations with Magnet Assisted Transfection (MATra-si) reagent for siRNAs (IBA GmbH, Göttingen, Germany) according to the manufacturers' protocols. Briefly, the siRNA was mixed with MATra-si reagent composed of supermagnetic ironoxide nanoparticles and incubated for 20 min. A complex was formed which was added to the cells to be transfected. A strong magnetic force was applied beneath the cells for 15 min. This forced the complex to be drawn into the cells by the magnetic field, and as a result the siRNA was delivered directly into the cytosol. For carrier free transfection experiments, cpRNAs were simply added to cells lines MG63, HOB, HCT116, fibroblasts and keratinocytes at 50 nM concentrations. Cells were also transfected with negative control siRNA (scrambled sequence) and cells left untreated were taken as controls. Each transfection was performed in triplicate. Post transfection, cells were incubated for 24 hours.

For the experiments involving lysosomotropic agent chloroquine (CQ), CQ was first neutralized to pH 7.4 using 1 M NaOH prior to use. MG63 cells were transfected with dT₂, dT₅, dT₈, (dT₅)₂, as(dT₅), and bl(dT₅) siRNA in presence or absence of 100 µM CQ or using MATra-si and incubated for 24 h. Similar experiments were performed for other cpRNAs namely, dA₅, dT₅, dG₅, (dT₅)₂, rA₅ and dC₅. For the control experiment, cells were either left untreated or transfected with negative control siRNA (scrambled sequence). Each transfection experiment was performed in triplicate.

### Total RNA samples

After performing transfection experiments for 24 h, 48 h and 72 h respectively, total RNA was isolated from cells by adding 500 µl of lysis buffer (Qiagen, Germany), followed by homogenization of cell lysates using QIAshredder (Qiagen, Germany). RNA was extracted from cell lysates by RNeasy Mini Kit (Qiagen, Germany). All RNA samples were treated with DNase using TURBO-DNAfree (Ambion, USA). Agilent 2100 BioAnalyzer (Agilent Technologies, Palo Alto, CA) was used to confirm high quality of all RNA samples, which reports an RNA Integrity Number (RIN) that takes into account the entire electrophoretic RNA trace produced in the analysis. A RIN of greater than or equal to seven indicates the RNA is suitable for high stringency applications.

The NanoDrop ND-1000 (NanoDrop Technologies, Wilmington, DE) was used to determine the concentrations, with resulting OD 260/280 ratios between 1.95-2.03.

### Real-time RT-PCR experiment

cDNA synthesis was performed in triplicate using total RNA reverse transcribed using High Capacity cDNA reverse transcription kit (Applied Biosystems, USA), with non-template control added to ensure a lack of signal in assay background. Reactions were incubated on a 96-well Applied Biosystems 9800 Fast Thermal Cycler PCR System at 37 °C for 60 min followed by 95 °C for 5 min. The real-time PCR reactions were carried out with 10 µl of 2x TaqMan® Universal PCR Master Mix, no AmpErase® UNG (Applied Biosystems, USA), 9 µl diluted cDNA, and 1 µl of TaqMan gene specific assay mix in a 20 µl final reaction volume. Reference gene beta-actin (ACTB) (Applied Biosystems, USA) was selected as control for normalization of TaqMan data. The assay included a no-template control, a standard curve of five serial dilution points (in steps of 3-fold) of cDNA mixture. Probes specific for IFN-α (Hs01022060_m1), IFN-β (Hs01077958_s1), IFN-γ (Hs00985251_m1), ACTB(Hs01060665_g1) and GAPDH (Hs02758991_g1) were purchased from Applied Biosystems. The amplification was carried out using the 7500 Fast Real-Time PCR System (Applied Biosystems, USA) using a 40-cycle program. The 7500 software automatically calculates raw Ct (cycle threshold) values. Data from samples with a Ct value equal to or below 35 were further analyzed. Samples were normalized relative to endogenous control and differences in cycle number thresholds were calculated using comparative quantitation 2^{-ΔΔCT} method (also called the ΔΔCT method), commonly used for analyzing siRNA induced gene knockdown efficiency.

The formulas used to calculate gene knockdown were as follows:
First, the ΔCT was calculated as the mean cycle threshold for the target gene minus the mean cycle thresholds for the endogenous controls ACTB, each performed in triplicates: ΔCT = CT (target gene) - CT (endogenous control). Secondly, the ΔΔCT was calculated as the ΔCT of the target minus the ΔCT of negative control (NC): ΔΔCT = ΔCT (target) - ΔCT (NC). Thereafter, the percentage of knockdown of target gene was calculated as: Fold change = 2^{-ΔΔCT}, then percentage of knockdown: = 100 × (1-fold change)

**Western blot analysis:** After 48 hours of transfection, cells were washed twice with ice-cold PBS and lysed in RIPA lysis buffer (50 mM Tris-HCI, pH 8.0, with 150 mM NaCl, 1.0% Igepal CA-630 (NP-40), 0.5% sodiumdeoxycholate, 0.1% SDS, and 1.0% protease inhibitor cocktail from SIGMA-ALDRICH®). Lysates were incubated on ice for 30 minutes and centrifuged at 10,000 rpm for 20 minutes to collect supernatant. Coomassie Plus - The Better Bradford Assay™ Reagent (Thermo Scientific) was used to measure protein concentrations. Thereafter, 20 µg of soluble protein was separated by SDS-PAGE and transferred to polyvinylidene difluoride membrane (Millipore). Primary antibodies against *GAPDH* (1:1000 dilution), β-catenin (1:1000 dilution; SIGMA-ALDRICH®), β-actin (1:1000; Cell Signaling Technology®) were used to probe the protein bands. Anti-Rabbit-HRP conjugated secondary antibodies (1:3000 dilution; R&D Systems®) were used to detect the primary antibodies, followed by the target protein visualization with EMD Millipore Immobilon™ Western Chemiluminescent HRP Substrate (ECL). Images were acquired using LI-COR Odyssey® Fc Dual-Mode Imaging system (LI-COR®Biosciences) and Image Studio Software.

### Statistical Analysis

The Student's *t*-test was used to determine statistical differences between pairs of groups. Two-way analysis of variance (ANOVA) was used to evaluate the statistical significance for comparisons within groups. p < 0.05 (two-sided) was considered as statistically significant. Data were analyzed using GraphPad Prism software package (version 6.0).

### RNAi activity of asymmetric siRNA

It was then explored if selective destabilization could influence RNAi activity because selective strand recruitment into RISC could improve this activity. To address this idea, a cell-based assay with human osteosarcoma cells (MG63) in which cellular components were present in physiologically relevant concentrations. As a model gene target, the housekeeping gene GAPDH (glyceraldehyde 3-phosphate dehydrogenase) was chosen, and RNAi activity was measured using quantitative RT-PCR with β-actin as the internal standard. Transfection experiments were performed with Magnet Assisted Transfection reagent (MATra-si) because this method carries reduced toxicity and does not rely on the conventional endocytosis mechanism generally observed with cationic polymer/lipid-based reagents.

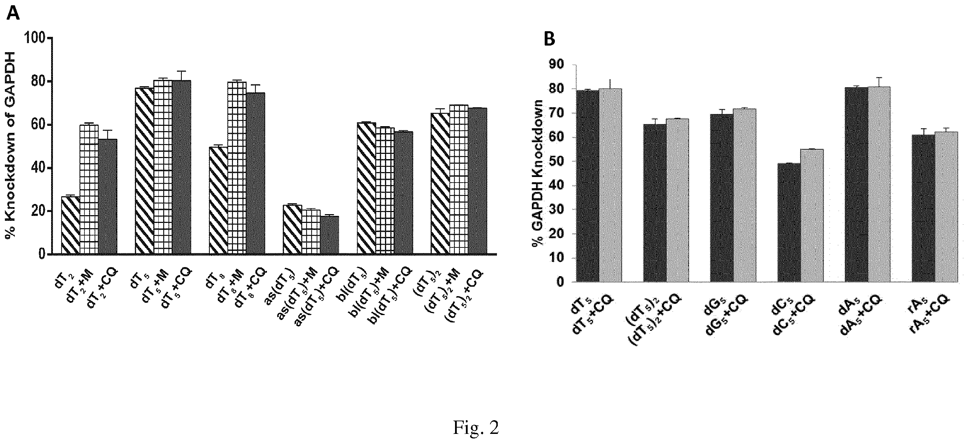

These experiments showed that an increase in overhang length to five or eight nt indeed resulted in superior gene-silencing activity (80 % gene knockdown) compared to canonical siRNA (60 % gene knockdown; Fig. 2A). This 20 % enhancement was attributed to the thermodynamic asymmetry of cpRNA resulting in preferential recruitment of antisense strand to RISC as compared to the canonical siRNA. Interestingly, when the symmetrical siRNA (dT₅)₂ was tested, a 10 % enhancement in activity (70 % gene knockdown) compared to canonical siRNA was seen. Though this activity was lower than cpRNA as anticipated, the enhancement is presumably due to easier unwinding of this siRNA over canonical siRNA.

### Cell-penetrating siRNA

Carrier-free transfection experiments were then performed using siRNA with different overhang lengths (dT₂, dT₅, dT₈ and (dT₅)₂). Surprisingly, these experiments showed that the dT₅-modified siRNA sequence resulted in 80 % gene knockdown (similar to MATra-si-based transfection) whereas dT₂- and dT₈-modified siRNA showed only modest 28 % and 50 % knockdown values, respectively (Fig. 2A). Symmetrical (dT₅)₂ modification, on the other hand, resulted in 65 % gene knockdown, which was comparable to outcomes with the MATra-si-based technique (70 %). This finding suggested that a siRNA design with a 5-nt overhang length (dT₅) performed all of the important steps for a siRNA-based drug, from cellular uptake to endosomal escape to selective RISC recruitment.

To further confirm the selectivity in RISC recruitment as a result of specific modification at the 3'-end, we tested *GAPDH-*siRNA where the dT₅ nts were incorporated at the 3'-end of antisense strand as(dT₅). This modification resulted in sharp decline in mRNA knockdown (20%) validating our hypothesis. Noteworthy, when we tested the blunt ended siRNA, bl(dT₅), where the 3'-dT₂ present at antisense strand was removed while retaining the dT₅ at the 3'-end of sense strand (Table-3), we observed a 20% decrease in activity (60% gene knockdown). These results reveal the critical parameters for RISC recruitment of siRNA.

We therefore designated siRNA with the 5-nt overhang design as cell penetrating siRNA or cpRNA. Cellular uptake of cpRNA was also confirmed by performing transfection experiments using Cy3 labeled cpRNA.

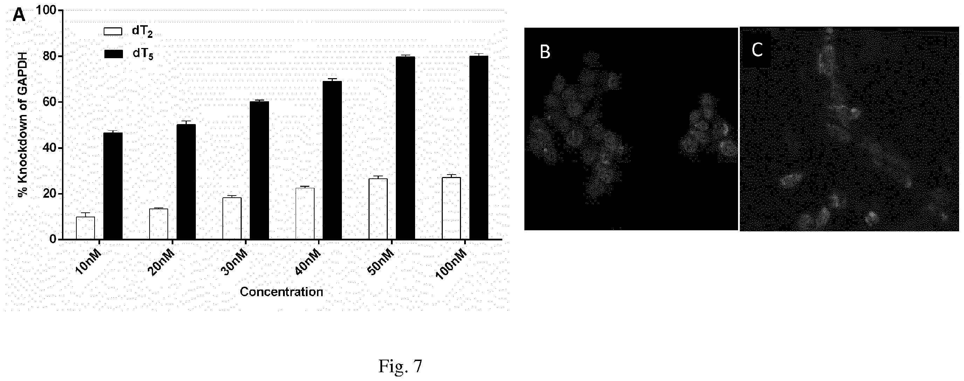

Additionally, we performed a concentration gradient study of siRNA and cpRNA (10 nM-100 nM) to determine the optimum concentration of siRNA for the transfection study. We observed highest gene knockdown (28% for canonical siRNA and 80% for dT₅ cpRNA) at the concentration of 50 nM after 24 h of incubation, which did not further increase when the concentration was increased to 100 nM (Fig. 7A). This experiment also demonstrated concentration dependent gene knockdown using cpRNA.

This intriguing result raised several important questions. (1) Is this phenomenon the result of better cellular uptake or of early release from the endosomal compartment, the sensitive region of the cell where RNA is generally trapped and degraded? (2) Is it specific to overhangs with a deoxyribose or ribose nt? (3) Is it specific to overhangs of a particular nt type (A, T, G, or C)? (4) Is it specific to a particular siRNA sequence? (5) Is it an energy-dependent process? (6) Do such modifications induce cytotoxicity or immune activation? Finally, (7) is it applicable to all cell types?

A series of experiments were performed to address these important questions. The carrier-free transfection experiment was repeated in presence of chloroquine, a lysosomotropic agent known to break the cellular endosome. To our surprise, this experiment yielded compelling evidence that cells take up all siRNA types (including canonical siRNA) but sequester them within the endosome. There, the siRNA molecules lose their activity, with the exception of siRNA bearing the 5-nt overhang (Fig. 2A).

Different purine and pyrimidine sequences in 5-nt repeats were then tested. The advantage of using such repeats is minimizing the possibility of sequence homology within the siRNA sequence (preventing secondary structures) and also limiting immune activation that is specific to dinucleotide repeats (e.g., CpG). Among different 5-nt candidates tested, siRNA with dA₅ and dT₅ overhangs demonstrated the highest RNAi activity (Fig. 2B). Of note, the ribose analogue rA₅ showed only 60 % knockdown, as compared to dA₅ (80 %), suggesting the presence of a specific enzymatic bias for deoxyribose over ribose nucleotides within the RISC complex. This result could also arise from differences in enzymatic degradation of single-stranded DNA versus single-stranded RNA.

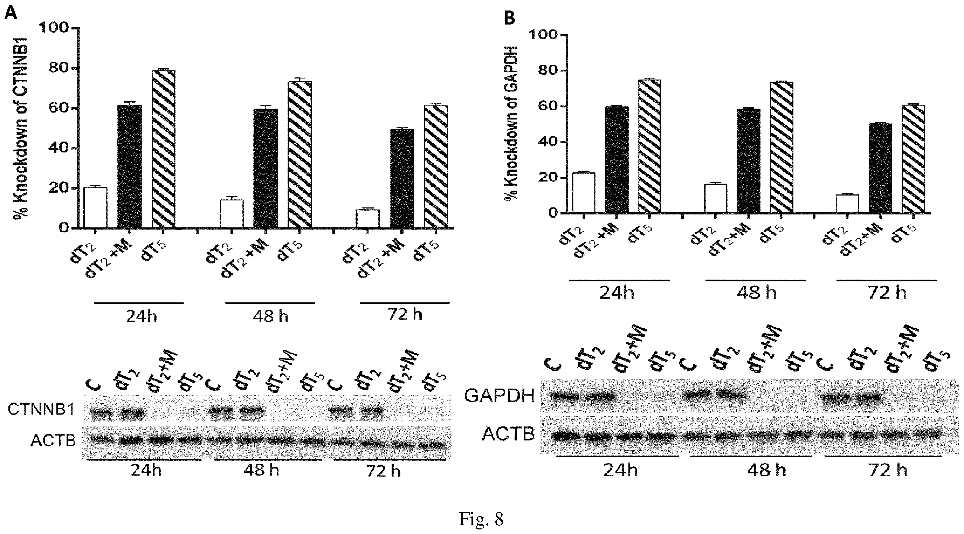

To further validate our results, we tested the gene knockdown efficiency of another gene *CTNNB1* (β-catenin), which is expressed in MG63 cells. We performed a time course experiment (24, 48 and 72 h) by targeting *CTNNB1* and *GAPDH* in MG63 cells and quantified the mRNA and protein levels using qRT-PCR and Western blot experiments respectively (Fig. 8). These experiments confirmed that cpRNA had higher gene knockdown efficiency for over 72 hrs without any transfection reagent as compared with that of canonical siRNA with transfection reagent (Fig. 8A, 8B). This result was also profound at the protein level, which showed complete knockout of *CTNNB1* and *GAPDH* proteins at 48 h with minor expression levels at 24 and 72 h.

### Versatility of cpRNA RNAi activity

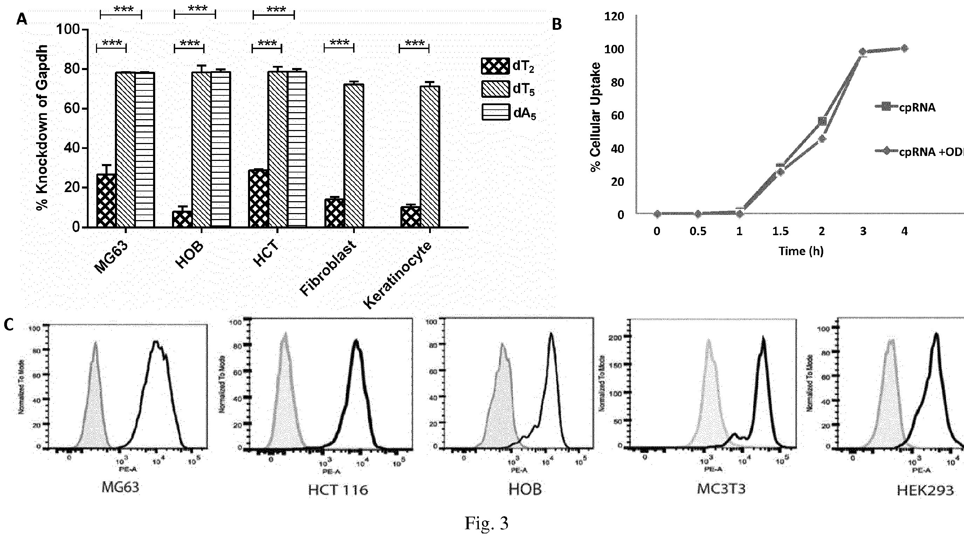

We then focused on the transfection efficiency of cpRNA in different cell types because transfection with different carriers generally works efficiently for fast-dividing cancer cells but not for hard-to-transfect primary cells. To test the transfection system with different cell types, human primary osteoblasts (HOB) were chosen as model primary cells; and human colon carcinoma cells (HCT116) and MG63 as model cancer cells. These experiments clearly demonstrated that cpRNA resulted in a 3-4-fold higher knockdown efficiency compared to canonical siRNA (Fig. 3A). This unique RNA design retained identical gene knockdown (80 %) capability in different cell types, even as canonical siRNA exhibited only limited activity in primary cells (Fig. 3A).

To quantify cellular uptake in different cells, flow cytometry experiments with were performed with different cell types using Cy3-labeled cpRNA where Cy3 was incorporated at the 5'-end of the sense strand with the 3'-dT₅ overhang. These experiments confirmed near quantitative cellular uptake in almost all cells tested (Fig. 3C). This Cy3-modified cpRNA also showed 80 % gene knockdown in MG63 cells, suggesting that blocking 5'-phosphorylation of the sense strand (inhibiting sense recruitment in RISC) did not further improve RNAi activity (data not shown). It also verified that hydrophobic modification at the 5'-end of the sense strand in cpRNA was well tolerated without any loss of activity.

These experiments showed that cpRNA having five nucleotide overhangs perform all necessary biochemical steps necessary for RNAi activity. These steps include, (1) cellular uptake, (2) endosomal escape, and (3) selective RISC recruitment. These results are indicated in Figs. 2 and 3.

### Cellular uptake of cpRNA is an energy dependent process

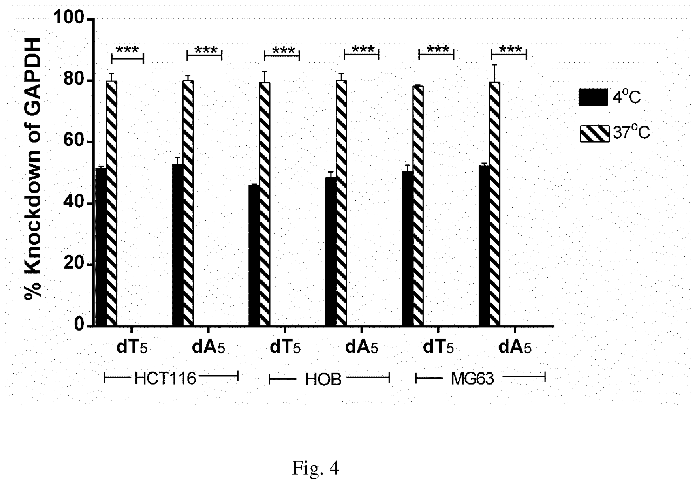

To determine, if cellular uptake of cpRNA is an energy dependent endocytosis process, transfection experiments were performed at 4 °C and 37 °C. Briefly, HCT116, HOB and MG63 cells were plated and incubated with cpRNA (dT₅ or dA₅ or scrambled siRNA sequence) at 4 °C or 37 °C for 4 h. After 4 h, medium was replaced with fresh medium and the cells were incubated for additional 24 h. Untreated cells were used as controls. Each transfection was performed in triplicate. These experiments clearly showed that cellular uptake of cpRNA is an energy dependent process. Cellular uptake using these siRNA sequences was reduced by nearly 30 % when experiments were performed at 4 °C, see Fig. 4.

### Mechanism of cellular penetration of cpRNA

Next, it was investigated if the cellular uptake mechanism of cpRNA occured by endocytosis. Transfection experiments were performed with dT₅-modified cpRNA at 4 °C and found lower RNAi activity (50% gene knockdown) compared to activity at 37 °C (80 %), suggesting an energy dependent endocytosis process for cellular uptake (Fig. 4). This result indicated the presence of an exquisitely sensitive, ubiquitous cell surface receptor that bound and internalized double-stranded RNA. Upon endocytosis, the 5-nt overhang length participated in an unknown endosomal escape mechanism that promoted cytosol transport of RNA. This unique mechanism was independent of nt type because every siRNA sequence with a 5-nt length showed identical efficiency compared to a chloroquine - based transfection (Fig. 2B). One possible candidate for such a receptor is the highly upregulated Toll-like receptor (TLR)-3 that together with TLR7, TLR8, or TLR9 has been implicated in recognizing extracellular RNA and for initiating innate immune activation of viral and synthetic RNAs. TLR-7, -8 and -9, selectively bind single-stranded DNA or RNA sequences with TLR-7 and -8 preferentially bind U and G rich sequence while TLR-9 bind CpG nt repeats. TLR3, on the other hand, nonspecifically binds the 21mer RNA duplex after dimerization of the receptor to form a 2:1 TLR3-RNA complex. This receptor is ubiquitously expressed on the cell surface and within endolysosomal compartments of almost all types of mammalian cells.

### Flow cytometry experiments showed efficient cellular uptake of cpRNA

To determine the amount of cellular uptake of cpRNA by flow cytometry, Cy3 labeled cpRNA (Table 3) was used for transfection experiments using different types of cells (MG63, HOB, HCT116, HEK293 and MC3T3). Cells were seeded at a density of 1×10⁵ cells per well and cultured for 24 hours. Thereafter, cells were transfected with 50 nM Cy3-cpRNA, with control wells left untreated. Post-transfection, cells were incubated for further 24 h at 37 °C, trypsinised and washed with PBS containing 2 mM EDTA and 0.5 % human serum albumin. The cells were then suspended in PBS and subjected to fluorescence analysis on a FACSCanto II (BD Biosciences) BioVis platform, scilife laboratory. The images were analyzed using FlowJo software (version 7.6; TreeStar, Ashland, OR). These experiments showed that Cy3 labeled cpRNA was quantitatively taken up by all the cells, see Fig. 3C.

To determine cpRNA cellular uptake kinetics, FACS experiments were repeated as mentioned above using MG63 cells, where Cy3-cpRNA was treated for different time points. Briefly, Cy3-cpRNA (50 nM) was added to cells, followed by incubation for 0.5 h, 1 h, 1.5 h, 2 h, 3 h and 4 h respectively. The cells were subsequently trypsinised and analyzed by FACS as mentioned above. These experiments showed that cellular uptake was initiated after 1 h and finished within next 2 h (Fig. 3B).

To perform TLR3 blocking studies, seeded MG63 cells were incubated with 600 nM 3'-FITC labeled ODN2006 for 1 h, followed by addition of 50 nM cpRNA. Cells were incubated for additional 0.5 h, 1 h, 1.5 h, 2 h, 3 h and 4 h respectively, trypsinised and analyzed by FACS analysis as mentioned above. These experiments showed that cellular uptake of cpRNA is not analogus to viral RNA or other larger double stranded RNA. Addition of ODN2006 generally inhibit cellular uptake of viral RNA, which was not observed with cpRNA (Fig. 3B).

To verify if the uptake mechanism of cpRNA was analogous to viral RNA and the RNA mimic polyriboinosinic:polyribocytidylic acid or poly(I:C), blocking studies were performed using the Btype single-stranded DNA sequence ODN2006 which share a common uptake receptor. This synthetic phosphorothioate modified oligo, though initially identified as a TLR9 agonist, blocks cellular uptake of viral RNA and poly(I:C) by preferentially competing with the common RNA uptake receptor. Blocking experiment were performed by pre-incubating the cells with FITC labeled ODN2006 for 1 h, followed by the addition of Cy3-labeled cpRNA. The blocking studies and cellular uptake was monitored using FACS analysis at different time points for up to 4 h. As a control experiment, the uptake kinetics was performed in the absence of FITC labeled ODN2006. Interestingly, these experiments demonstrated that cpRNA did not follow the same mechanism that viral RNA or poly(I:C); addition of FITC-ODN2006 did not inhibit uptake of cpRNA (Fig. 3B). Furthermore, unlike poly(I:C), which exhibited fast uptake kinetics (within 5 min), cellular uptake of cpRNA was initiated after ∼1 h of incubation at 37 °C, which subsequently completed in the next 2 h. These experiments verified the involvement of an alternate uptake mechanism and indicated the presence of a ubiquitous RNA capture receptor that required ∼1 h of maturation time before the endocytosis process could initiate.

### Cell proliferation assay indicating absence of cytotoxicity of cpRNA

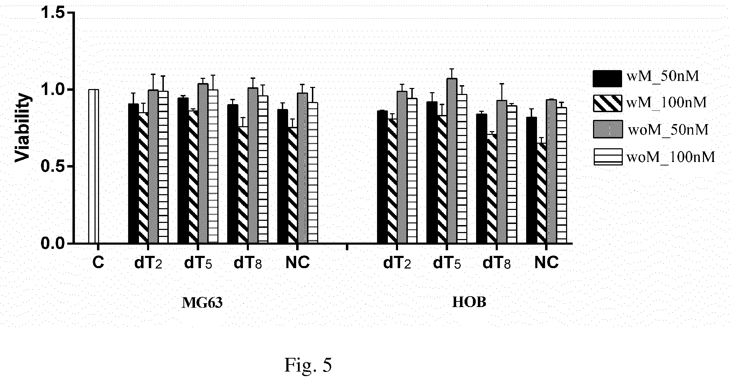

Cell proliferation was assessed by MTS (3-(4,5-dimethylthiazol-2-yl)-5-(3-carboxymethoxyphenyl)-2-(4-sulfophenyl)-2H-tetrazolium, inner salt) reagent assay: MG63 cells were seeded at a density of 35 000 cells in 24-well culture plates, and cultured for 24 hours as mentioned above. Cells were transfected with 50 nM or 100 nM concentrations of cpRNA and negative control siRNAs with or without MATra-si, while control wells were left untreated. Each transfection experiment was performed in triplicate. Post-transfection, cells were incubated for 24 hours at 37 °C. The viable cells were evaluated by MTS assay, using CellTiter 96®AQueousOne Solution Cell Proliferation Assay (Promega, USA) according to the manufacturer's protocol. The enzymatic reduction in MTS to formazan was quantified by an ELISA plate reader (Thermo Scientific) at 490 nm. This experiment shows that all modified siRNA having different overhang lengths do not induce any cytotoxicity (Fig. 5).

Thus, the role of siRNA topography on immune activation and cellular toxicity was investigated because increased RNA length has been reported to cause toxicity with increased interferon (IPN)-β expression. toxicity studies were performed using the MTS assay with two concentrations of GAPDH siRNA (50 and 100 nM) with different overhang lengths (dT₂, dT₅, and dT₈) in MG63 and HOB cells and compared it with the scrambled siRNA sequence (Fig. 5). These experiments clearly showed that overhangs of different lengths do not impose toxicity on cancer cells or primary cells at high concentrations (100 nM). MATra-si-based transfection, however, resulted in slightly higher toxicity as compared to carrier-free experiments at 100 nM siRNA concentrations, which is clearly because of higher amounts of transfection agent, consistent with previous observations on carrier toxicity.

### Interferon assay showed no immune activation of modified RNA

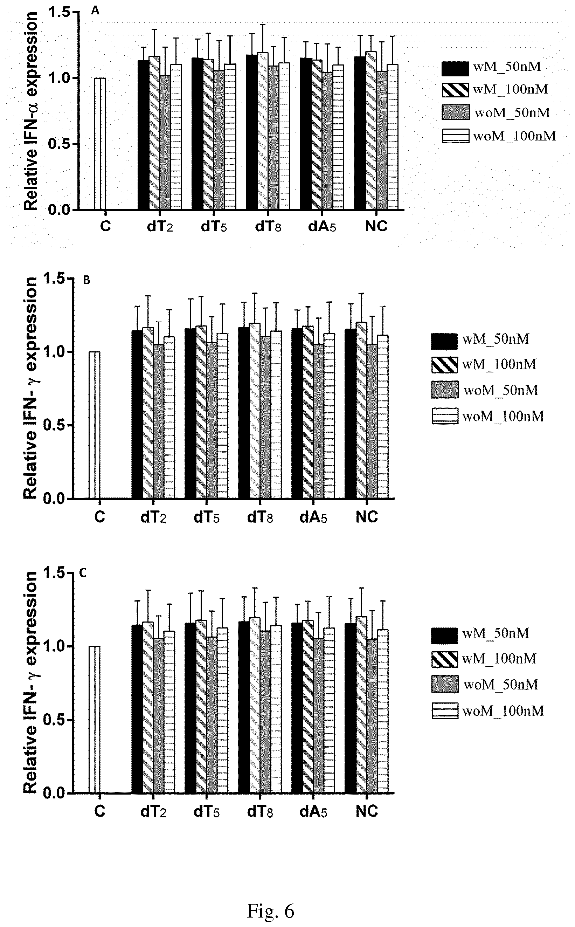

MG63 cells were transfected with 50 nM or 100 nM concentrations of cpRNA and negative control siRNAs with or without MATra-si as mentioned above. The control wells were left untreated. Each transfection experiment was performed in triplicates. After 24 h, interferon inductions were tested by RT-PCR experiments using primers specific for interferon α, β and γ. These experiments demonstrated that modified siRNAs having different overhang lengths do not trigger immune reaction (Fig. 6).

Thus, because cellular stress mainly originates from maturation of the RNA-TLR complex in the endosome or activation of RIG-1 in the cytoplasm, we investigated immune activation and IFN response using cpRNA and a scrambled sequence. The levels of Type-I (IFN-α and β) and Type-II (IFN-y) IFN were tested via quantitative RT-PCR. IFN-α, β, and γ are activated by siRNA in different cells, *in vitro* and *in vivo.* Of note, MG63 cells are 300 to 500 fold more sensitive to IFN-β induction than other cell types such as HeLa. In these experiments, cpRNA showed no significant IFN response compared to cell-alone control or the scrambled sequence (Fig. 6). This finding is presumably due to the fast release of the cpRNA from the endolysosome compartment, preventing TLR maturation, which is a key step in initiating the TLR-mediated IFN response.

### Fluorescence Microscopy

Cellular uptake experiments were performed by plating cells (1×10⁴) in an 8 well chamber slide. At 70 % confluency, the cells were transfected with 50 nM Cy3-cpRNA and incubated at 37 °C for 24 h. Control wells were left untreated. Each transfection was performed in triplicate. To evaluate the distribution of fluorescently labeled cpRNA, cells were fixed with methanol-acetone and washed with PBS. The slides were mounted using Vectashield mounting medium containing 4',6-diamidino-2-phenylindole (DAPI) (Vector Labs, Burlingame, CA) to stain the nuclei of cells. Images were captured with an Axiolmager Z1 microscope equipped with an Apotome (Carl Zeiss AB, Stockholm, Sweden).

### GFP knockdown in MG-63 cells

MG63 cells constitutively expressing green fluorescent protein (GFP) were seeded (1×10⁴) in an 8 well chamber slide. After 24 h the cells were transfected with 50 nM of GFP-cpRNA or GFP-siRNA as mentioned earlier. Each transfection was performed in triplicate. Control wells were left untreated. The cells were then incubated for 48 h.

To evaluate gene knockdown (GFP levels), cells were fixed with methanol-acetone and washed with PBS. Slides were mounted using Vectashield mounting medium containing DAPI to stain the nuclei in all the samples. Images were captured with an Axiolmager Z1 microscope equipped with an Apotome (Carl Zeiss AB, Stockholm, Sweden).

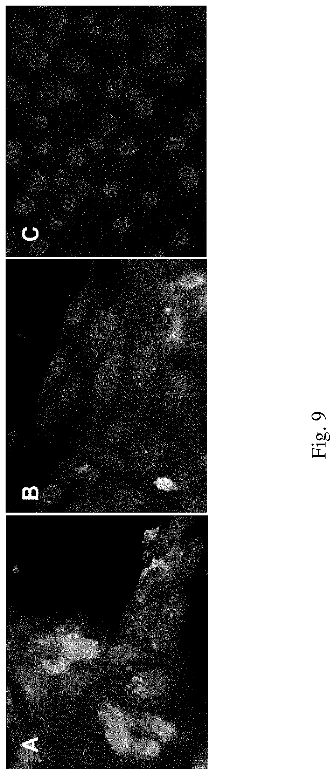

Upon incubating these GFP-expressing MG63 cells with GFP-cpRNA (50 nM), almost complete knockdown of green fluorescence was observed in 48 h (Fig. 9C). With GFP-siRNA control, on the other hand, GFP expression could be observed (Fig. 9B), exemplifying the unique property of cpRNA.

### Hyaluronan-cpRNA conjugation and in vitro evaluation

We explored HA-cpRNA conjugation method exploiting hydrazone linkages. For this purpose, we incorporated Uracil nucleoside at the 3'end of the sense strand of our modified cpRNA (see aldehyde-modified GAPDH cpRNA sequences above). The ribosugar in the terminal nucleotide was oxidized using sodium per iodate (NaIO₄) to obtain aldehyde modified cpRNA. For this purpose, 50µl (50 nmol) of the 100 µM cpRNA was taken in a sterile eppendorf tube and 5 µl of 50 nmol sodium per iodate (NaIO₄) was added to the solution. After this cpRNA was purified using ethanol precipitation and further purified by desalting column and lyophilized. The purified cpRNA was resuspended in RNAse free water and mixed with 50µl of hydrazide modified HA (200 nmol with 10% modification) and incubated for 1 h. The conjugation was verified by performing gel electrophoresis using 20% polyacrylamide gel (Fig. 10A).

In order to perform the functional evaluation of HA-cpRNA conjugate we selected C2C12 cell lines. The siRNA was selected to target house keeping gene GAPDH. The gene knockdown levels were evaluated using qPCR analysis.

After careful optimization, we were able to develop HA-cpRNA conjugate as could be demonstrated by gel electrophoresis assay (Figure 10A). The conjugated product because of being high molecular weight moved slowly in the gel as compared to the unconjugated siRNA or cpRNA. Comparison of gene knockdown between different groups also showed that conjugation strategy is not detrimental for its bioactivity (Figure 10B). The HA-cpRNA conjugate at both 50 and 100 nM concentrations gave similar gene knockdown as compared to the siRNA-Lipo control. This result is contrary to general observation of HA-siRNA conjugates which do not show gene knockdown without using cationic polymer such as PEI (Bioconjugate Chem. 2013, 24, 1201-1209).

### Conclusions

The double-stranded siRNA molecule of the invention, cpRNA, represents the first example of transfection agent-free cellular delivery of active siRNA using unmodified nucleotides. Based on the presented findings, any siRNA molecule, regardless of sequence, can be transformed into a self-deliverable drug by incorporating deoxy nucleotides like A or T. This RNA design demonstrated efficient cellular uptake and endosomal escape with 3-4-fold higher bioactivity than conventional siRNA when used without any carrier molecule. This efficiency was demonstrated in cancer cells, and hard-to-transfect primary human osteoblasts. Further findings suggested that the higher bioactivity of this siRNA design presumably arises from preferential strand selection within the RISC complex, resulting from the selective destabilization of the 5'-end of the antisense strand. This cpRNA also induced none of the toxicity or immunogenicity associated with larger siRNA molecules.

### REFERENCES

[1] Grundberg et al., PLoS genetics 7, e1001279 (2011).
[2] Grundberg et al., Genome research 19, 1942 (2009).

## Claims

1. A double-stranded small interfering ribonucleic acid (siRNA) molecule comprising a sense RNA strand and an antisense RNA strand, wherein said sense strand has a 3'-overhang of from four to eight nucleotides, of which at least one nucleotide is a non-ribonucleotide, wherein said antisense strand has a 3'-overhang that is shorter than said 3'-overhang of said sense strand, and wherein a double-stranded portion of said double-stranded siRNA molecule has a length of at least 19 base pairs.

2. The double-stranded siRNA molecule according to claim 1, wherein said antisense strand has a 3'-overhang of at least two nucleotides, or wherein said antisense strand has a 3'-overhang of from two to five nucleotides.

3. The double-stranded siRNA molecule according to claim 1 or claim 2, wherein the non-ribonucleotide is a deoxynucleotide.

4. The double-stranded siRNA molecule according to any one of claims 1 to 3, wherein all of said nucleotides in said 3'-overhang of the sense strand are non-ribonucleotides, or wherein all of the non-ribonucleotides in the 3'-overhang of the sense strand are deoxynucleotides.

5. The double-stranded siRNA molecule according to any one of claims 1 to 4, wherein at least one nucleotide in said 3'-overhang of said antisense strand is a non-ribonucleotide, optionally wherein the at least one non-ribonucleotide in said 3'-overhang of said antisense strand is a deoxynucleotide.

6. A cell transfection composition comprising a double-stranded small interfering ribonucleic acid (siRNA) molecule, wherein said double-stranded siRNA molecule comprises a sense RNA strand and an antisense RNA strand, wherein said sense strand has a 3'-overhang of from four to eight nucleotides, and wherein said composition does not comprise a cationic agent.

7. A cell transfection composition comprising a double-stranded small interfering ribonucleic acid (siRNA) molecule, wherein said double-stranded siRNA molecule is as defined in any one of claims 1 to 5.

8. The composition according to claim 6 or claim 7, wherein said composition comprises a carrier.

9. The composition according to any one of claims 6 to 8, wherein said composition is a pharmaceutical composition comprising a pharmaceutically acceptable diluent.

10. A method of target-specific ribonucleic acid (RNA) interference in a cell comprising contacting in vitro said cell with a double-stranded small interfering RNA (siRNA) molecule in the absence of a cationic agent, wherein said double-stranded siRNA molecule comprises a sense RNA strand and an antisense RNA strand, wherein said sense strand has a 3'-overhang of from four to eight nucleotides, and wherein at least a portion of said antisense strand of said double stranded siRNA molecule has a nucleotide sequence that is complementary to a nucleotide sequence of a target RNA sequence.

11. A method of target-specific ribonucleic acid (RNA) interference in a cell comprising contacting in vitro said cell with a double-stranded small interfering RNA (siRNA) molecule, wherein said double-stranded siRNA molecule is as defined in any one of claims 1 to 5, and wherein at least a portion of said antisense strand of said double stranded siRNA molecule has a nucleotide sequence that is complementary to a nucleotide sequence of a target RNA sequence.

12. The method according to claim 10 or claim 11, wherein said cell is contacted with said double-stranded siRNA molecule in the presence of a carrier.

13. A double-stranded small interfering ribonucleic acid (siRNA) molecule for use in the treatment of a disease by sequence-specific knockdown of a target RNA sequence, wherein said double-stranded siRNA molecule comprises a sense RNA strand and an antisense RNA strand, wherein said sense strand has a 3'-overhang of from four to eight nucleotides, wherein at least a portion of an antisense strand of said double-stranded siRNA molecule has a nucleotide sequence that is complementary to a nucleotide sequence of said target RNA sequence, and wherein said double-stranded siRNA molecule is administered to a subject in the absence of a cationic agent.

14. A double-stranded small interfering ribonucleic acid (siRNA) molecule for use in the treatment of a disease by sequence-specific knockdown of a target RNA sequence, wherein said double-stranded siRNA molecule is as defined in any one of claims 1 to 5, and wherein at least a portion of an antisense strand of said double-stranded siRNA molecule has a nucleotide sequence that is complementary to a nucleotide sequence of said target RNA sequence.

15. The double-stranded small interfering ribonucleic acid (siRNA) molecule for use according to claim 13 or claim 14, wherein said double-stranded siRNA molecule is administered with a carrier.

## Patentansprüche

1. Doppelsträngiges Small-Interfering-Ribonukleinsäure-(siRNA)-Molekül, umfassend einen Sense-RNA-Strang und einen Antisense-RNA-Strang, wobei der Sense-Strang einen 3'-Überhang von vier bis acht Nukleotiden aufweist, von denen mindestens ein Nukleotid ein Nicht-Ribonukleotid ist, wobei der Antisense-Strang einen 3'-Überhang aufweist, der kürzer ist als der 3'-Überhang des sense-Strangs, und wobei ein doppelsträngiger Abschnitt des doppelsträngigen siRNA-Moleküls eine Länge von mindestens 19 Basenpaaren aufweist.

2. Doppelsträngiges siRNA-Molekül nach Anspruch 1, wobei der Antisense-Strang einen 3'-Überhang von mindestens zwei Nukleotiden aufweist, oder wobei der Antisense-Strang einen 3'-Überhang von zwei bis fünf Nukleotiden aufweist.

3. Doppelsträngiges siRNA-Molekül nach Anspruch 1 oder Anspruch 2, wobei das Nicht-Ribonukleotid ein Desoxynukleotid ist.

4. Doppelsträngiges siRNA-Molekül nach einem der Ansprüche 1 bis 3, wobei alle Nukleotide im 3'-Überhang des Sense-Strangs Nicht-Ribonukleotide sind, oder wobei alle Nicht-Ribonukleotide im 3'-Überhang des Sense-Strangs Desoxynukleotide sind.

5. Doppelsträngiges siRNA-Molekül nach einem der Ansprüche 1 bis 4, wobei mindestens ein Nukleotid im 3'-Überhang des Antisense-Strangs ein Nicht-Ribonukleotid ist, gegebenenfalls wobei das mindestens eine Nicht-Ribonukleotid im 3'-Überhang des Antisense-Strangs ein Desoxynukleotid ist.

6. Zelltransfektionszusammensetzung, umfassend ein doppelsträngiges Small-Interfering-Ribonukleinsäure-(siRNA)-Molekül, wobei das doppelsträngige siRNA-Molekül einen sense-RNA-Strang und einen Antisense-RNA-Strang umfasst, wobei der sense-Strang einen 3'-Überhang von vier bis acht Nukleotiden aufweist, und wobei die Zusammensetzung kein kationisches Mittel umfasst.

7. Zelltransfektionszusammensetzung, umfassend ein doppelsträngiges Small-Interfering-Ribonukleinsäure-(siRNA)-Molekül, wobei das doppelsträngige siRNA-Molekül wie in einem der Ansprüche 1 bis 5 definiert ist.

8. Zusammensetzung nach Anspruch 6 oder Anspruch 7, wobei die Zusammensetzung einen Träger umfasst.

9. Zusammensetzung nach einem der Ansprüche 6 bis 8, wobei die Zusammensetzung eine pharmazeutische Zusammensetzung ist, die ein pharmazeutisch verträgliches Verdünnungsmittel umfasst.

10. Verfahren zur zielspezifischen Ribonukleinsäure (RNA)-Interferenz in einer Zelle, umfassend das in vitro Inkontaktbringen der Zelle mit einem doppelsträngigen Small-Interfering-Ribonukleinsäure-(siRNA)-Molekül in Abwesenheit eines kationischen Mittels, wobei das doppelsträngige siRNA-Molekül einen sense-RNA-Strang und einen antisense-RNA-Strang umfasst, wobei der Sense-Strang einen 3'-Überhang von vier bis acht Nukleotiden aufweist, und wobei mindestens ein Teil des Antisense-Strangs des doppelsträngigen siRNA-Moleküls eine Nukleotidsequenz aufweist, die komplementär zu einer Nukleotidsequenz einer Ziel-RNA-Sequenz ist.

11. Verfahren zur zielspezifischen Ribonukleinsäure (RNA)-Interferenz in einer Zelle, umfassend das in vitro Inkontaktbringen der Zelle mit einem doppelsträngigen Small-Interfering-Ribonukleinsäure-(siRNA)-Molekül, wobei das doppelsträngige siRNA-Molekül wie in einem der Ansprüche 1 bis 5 definiert ist, und wobei mindestens ein Teil des Antisense-Strangs des doppelsträngigen siRNA-Moleküls eine Nukleotidsequenz aufweist, die komplementär zu einer Nukleotidsequenz einer Ziel-RNA-Sequenz ist.

12. Verfahren nach Anspruch 10 oder Anspruch 11, wobei die Zelle mit dem doppelsträngigen siRNA-Molekül in Gegenwart eines Trägers kontaktiert wird.

13. Doppelsträngiges Small-Interfering-Ribonukleinsäure-(siRNA)-Molekül zur Verwendung bei der Behandlung einer Krankheit durch sequenz-spezifisches Zerlegen einer Ziel-RNA-Sequenz, wobei das doppelsträngige siRNA-Molekül einen Sense-RNA-Strang und einen Antisense-RNA-Strang umfasst, wobei der Sense-Strang einen 3'-Überhang von vier bis acht Nukleotiden aufweist, wobei mindestens ein Teil eines Antisense-Strangs des doppelsträngigen siRNA-Moleküls eine Nucleotidsequenz aufweist, die zu einer Nucleotidsequenz der Ziel-RNA-Sequenz komplementär ist, und wobei das doppelsträngige siRNA-Molekül einer Testperson in Abwesenheit eines kationischen Mittels verabreicht wird.

14. Doppelsträngiges Small-Interfering-Ribonukleinsäure-(siRNA)-Molekül zur Verwendung bei der Behandlung einer Erkrankung durch sequenz-spezifisches Zerlegen einer Ziel-RNA-Sequenz, wobei das doppelsträngige siRNA-Molekül wie in einem der Ansprüche 1 bis 5 definiert ist und wobei mindestens ein Teil eines Antisense-Strangs des doppelsträngigen siRNA-Moleküls eine Nukleotidsequenz aufweist, die komplementär zu einer Nukleotidsequenz der Ziel-RNA-Sequenz ist.

15. Doppelsträngiges Small-Interfering-Ribonukleinsäure (siRNA)-Molekül zur Verwendung nach Anspruch 13 oder Anspruch 14, wobei das doppelsträngige siRNA-Molekül mit einem Träger verabreicht wird.

## Revendications

1. Molécule de petit acide ribonucléique interférent (ARNsi) double brin comprenant un brin d'ARN sens et un brin d'ARN antisens, dans laquelle ledit brin sens comporte un porte-à-faux 3' de quatre à huit nucléotides, parmi lesquels au moins un nucléotide est un non-ribonucléotide, dans laquelle ledit brin antisens comporte un porte-à-faux 3' qui est plus court que ledit porte-à-faux 3' dudit brin sens, et dans laquelle une partie double brin de ladite molécule d'ARNsi double brin possède une longueur d'au moins 19 paires de bases.

2. Molécule d'ARNsi double brin selon la revendication 1, dans laquelle ledit brin antisens comporte un porte-à-faux 3' d'au moins deux nucléotides, ou dans laquelle ledit brin antisens comporte un porte-à-faux 3' de deux à cinq nucléotides.

3. Molécule d'ARNsi double brin selon la revendication 1 ou la revendication 2, dans laquelle le non-ribonucléotide est un désoxynucléotide.

4. Molécule d'ARNsi double brin selon l'une quelconque des revendications 1 à 3, dans laquelle l'ensemble desdits nucléotides dans ledit porte-à-faux 3' du brin sens sont des non-ribonucléotides, ou dans laquelle l'ensemble des non-ribonucléotides dans le porte-à-faux 3' du brin sens sont des désoxynucléotides.

5. Molécule d'ARNsi double brin selon l'une quelconque des revendications 1 à 4, dans laquelle au moins un nucléotide dans ledit porte-à-faux 3' dudit brin antisens est un non-ribonucléotide, facultativement dans laquelle le au moins un non-ribonucléotide dans ledit porte-à-faux 3' du brin antisens est un désoxynucléotide.

6. Composition de transfection cellulaire comprenant une molécule de petit acide ribonucléique interférent (ARNsi) double brin, dans laquelle ladite molécule d'ARNsi double brin comprend un brin d'ARN sens et un brin d'ARN antisens, dans laquelle ledit brin sens comporte un porte-à-faux 3' de quatre à huit nucléotides, et dans laquelle ladite composition ne comprend pas d'agent cationique.

7. Composition de transfection cellulaire comprenant une molécule de petit acide ribonucléique interférent (ARNsi) double brin, dans laquelle ladite molécule d'ARNsi double brin est telle que définie dans l'une quelconque des revendications 1 à 5.

8. Composition selon la revendication 6 ou la revendication 7, dans laquelle ladite composition comprend un véhicule.

9. Composition selon l'une quelconque des revendications 6 à 8, dans laquelle ladite composition est une composition pharmaceutique comprenant un diluant pharmaceutiquement acceptable.

10. Procédé d'interférence de l'acide ribonucléique (ARN) spécifique d'une cible dans une cellule comprenant la mise en contact *in vitro* de ladite cellule avec une molécule de petit ARN interférent (ARNsi) double brin en l'absence d'agent cationique, dans lequel ladite molécule d'ARNsi double brin comprend un brin d'ARN sens et un brin d'ARN antisens, dans lequel ledit brin sens comporte un porte-à-faux 3' de quatre à huit nucléotides, et dans lequel au moins une partie dudit brin antisens de ladite molécule d'ARNsi double brin possède une séquence de nucléotides qui est complémentaire à une séquence de nucléotides d'une séquence d'ARN cible.

11. Procédé d'interférence de l'acide ribonucléique (ARN) spécifique d'une cible dans une cellule comprenant la mise en contact *in vitro* de ladite cellule avec une molécule de petit ARN interférent (ARNsi) double brin, dans lequel ladite molécule d'ARNsi double brin est telle que définie dans l'une quelconque des revendications 1 à 5, et dans lequel au moins une partie dudit brin antisens de ladite molécule d'ARNsi double brin possède une séquence de nucléotides qui est complémentaire à une séquence de nucléotides d'une séquence d'ARN cible.

12. Procédé selon la revendication 10 ou la revendication 11, dans lequel ladite cellule est mise en contact avec ladite molécule d'ARNsi double brin en présence d'un véhicule.

13. Molécule de petit acide ribonucléique interférent (ARNsi) double brin destinée à être utilisée dans le traitement d'une maladie par une extinction spécifique de séquence d'une séquence d'ARN cible, dans laquelle ladite molécule d'ARNsi double brin comprend un brin d'ARN sens et un brin d'ARN antisens, dans laquelle ledit brin sens comporte un porte-à-faux 3' de quatre à huit nucléotides, dans laquelle au moins une partie d'un brin antisens de ladite molécule d'ARNsi double brin possède une séquence de nucléotides qui est complémentaire à une séquence de nucléotides de ladite séquence d'ARN cible, et dans laquelle ladite molécule d'ARNsi double brin est administrée à un sujet en l'absence d'agent cationique.

14. Molécule de petit acide ribonucléique interférent (ARNsi) double brin destinée à être utilisée dans le traitement d'une maladie par une extinction spécifique de séquence d'une séquence d'ARN cible, dans laquelle ladite molécule d'ARNsi double brin est telle que définie dans l'une quelconque des revendications 1 à 5, et dans laquelle au moins une partie d'un brin antisens de ladite molécule d'ARNsi double brin possède une séquence de nucléotides qui est complémentaire à une séquence de nucléotides de ladite séquence d'ARN cible.

15. Molécule de petit acide ribonucléique interférent (ARNsi) double brin destinée à être utilisée selon la revendication 13 ou la revendication 14, dans laquelle ladite molécule d'ARNsi double brin est administrée avec un véhicule.
